# EUROPEAN PATENT SPECIFICATION

(11) **EP 3 003 350 B1**
(45) Date of publication and mention of the grant of the patent: **21.02.2018**
(21) Application number: 14728872.4
(22) Date of filing: 27.05.2014
(51) Int. Cl.: A61K 38/17, C07K 16/28, C07K 14/47

(54) **TARGETED MODULATION OF MACROPHAGES**
GEZIELTE MODULATION VON MAKROPHAGEN
MODULATION CIBLÉE DE MACROPHAGES

(30) Priority: 27.05.2013 EP 13169381
(43) Date of publication of application: 13.04.2016
(73) Proprietor: Fraunhofer-Gesellschaft zur Förderung der angewandten Forschung e.V., 80686 München (DE); Rheinisch-Westfälische Technische Hochschule Aachen, 52062 Aachen (DE)
(72) Inventor: THEPEN, Theophilus, 52074 Aachen (DE); HRISTODOROV, Dmitrij, 52062 Aachen (DE); MLADENOV, Radoslav, 8049 Zürich (CH); BARTH, Stefan, 52074 Aachen (DE)
(74) Representative: dompatent von Kreisler Selting Werner - Partnerschaft von Patent- und Rechtsanwälten mbB
(86) International application number: PCT/EP2014/060914
(87) International publication number: WO 2014/191391

(56) References cited:
- WO-A1-2010/045584
- DMITRIJ HRISTODOROV ET AL: "Macrophage-targeted therapy: CD64-based immunotoxins for treatment of chronic inflammatory diseases", TOXINS, MOLECULAR DIVERSITY PRESERVATION INTERNATIONAL (MDPI) AG, CH, [Online] vol. 4, no. 9, 1 September 2012 (2012-09-01), pages 676-694, XP002692453, ISSN: 2072-6651, DOI: 10.3390/TOXINS4090676 Retrieved from the Internet: URL:http://www.mdpi.com/2072-6651/4/9/676> [retrieved on 2012-09-14]
- MATI FRIDKIN ET AL: "Tuftsin-AZT conjugate: potential macrophage targeting for AIDS therapy", JOURNAL OF PEPTIDE SCIENCE, vol. 11, no. 1, 1 January 2005 (2005-01-01), pages 37-44, XP055034404, ISSN: 1075-2617, DOI: 10.1002/psc.587

## Description

The present invention pertains to an immunomodulatory fusion protein (IFP) for converting pro-inflammatory M1 macrophages to anti-inflammatory M2 like phenotypes of macrophages the IFP, a polynucleotide encoding the immunomodulatory fusion protein (IFP) of the invention, a process of manufacturing the immunomodulatory fusion protein (IFP) of the invention, a vector comprising or consisting of the polynucleotide of the invention, a cell comprising or consisting of the vector of the invention, a medicament comprising or consisting of the immunomodulatory fusion protein (IFP) of the invention as well as the use of the immunomodulatory fusion protein (IFP) in the treatment of chronic inflammatory diseases.

Macrophages are key components of the innate immune system which play a principal role in the regulation of inflammation [1] as well as physiological processes such as tissue remodeling [2]. Differentiated macrophages and their precursors are versatile cells that can adapt to microenvironmental signals by altering their phenotype and function [3]. Although they have been studied for many years, it has only recently been shown that these cells comprise distinct subpopulations, known as classical M1 and alternative M2. Mirroring the nomenclature of Th1 cells, M1 macrophages are described as the pro-inflammatory subtype of macrophages induced by IFN-γ and LPS. They produce effector molecules (e.g., reactive oxygen species) and pro-inflammatory cytokines (e.g., IL-12, TNF-α and IL-6) and they trigger Th1 polarized responses [4]. M2 macrophages are further sub-divided into M2a (following exposure to IL-4 or IL-13), M2b (immunocomplexes and Toll-like receptors or IL-1b ligands) and M2c (induced by IL-10, TGF-β and glucocorticoids). All three subtypes have an anti-inflammatory phenotype characterized by IL-10^{high} (especially M2b+M2c), RELM-α^{high} (mouse only), CD206^{high} and IL-12^{low} (M2a) [5].

During normal inflammation, macrophages undergo dynamic switching between these polarization states. Whereas M1 macrophages are more abundant during the early stages and mediate clearance and the recruitment of other effector cells, M2 macrophages predominate towards the end of inflammation, promoting vascularization and new tissue formation [6, 7]. The course of inflammation is strongly dependent on this appropriately-balanced ratio of M1/M2 macrophages.

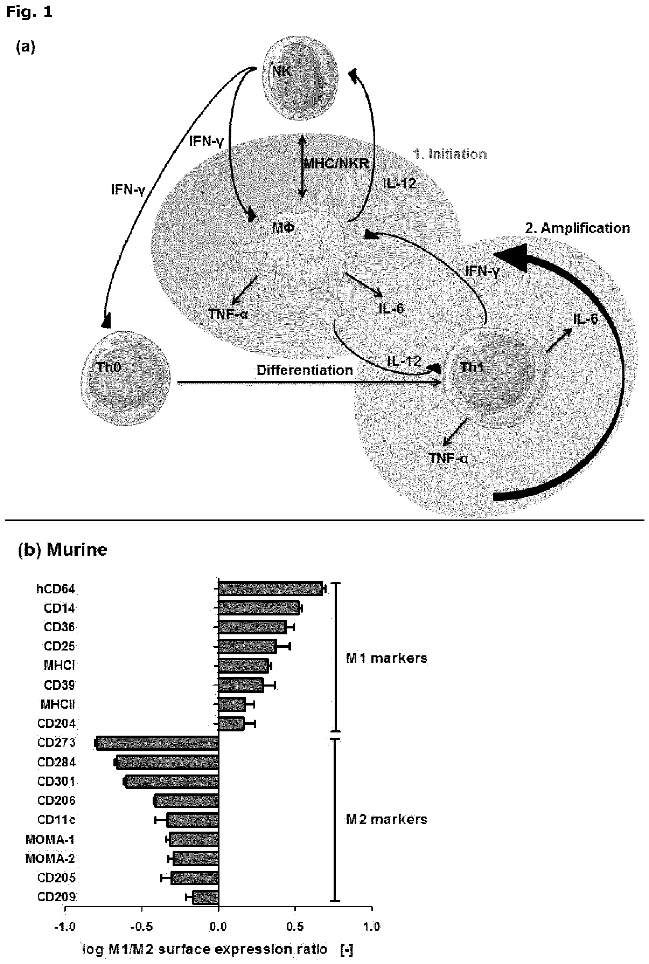

Failure to switch from the predominance of M1 to M2 may lead to the perpetuation and reinforcement of the pro-inflammatory environment in chronic inflammation (Fig. 1a). Therefore, M1 arrest may prevent the resolution of inflammation [8].

A disrupted M1/M2 ratio has been observed in several autoimmune and chronic inflammatory diseases, as well as metabolism-associated diseases such as diabetes and metabolic syndrome [9]. Adipose tissue macrophages (ATMs) from obese individuals have been shown to undergo a phenotypic shift from M2 (CD206⁺, CD301⁺, Arg1⁺) to M1 (NOS2⁺, CD11c⁺), producing pro-inflammatory cytokines such as IL-6, TNF-α, and IL-1β [10], thus antagonizing the effects of leptin and adiponectin [11]. Similar M1-associated pathology has been associated with cardiovascular diseases such as atherosclerosis [12], and autoimmune diseases such as rheumatoid arthritis [13], multiple sclerosis [14], systemic lupus erythematosus [15] and Crohn's disease [16].

The persistence of M1 macrophages during the initial inflammatory response can also prevent the resolution of inflammation in several chronic skin diseases. Reducing the number of pro-inflammatory M1 macrophages in diabetes-associated skin ulcerations can attenuate wound inflammation and promote wound closure. In human patients with chronic venous ulcers, the persistence of M1 macrophages induces the production of reactive oxygen species which cause DNA damage and lead to defective tissue repair. Activated (presumably M1) macrophages are also associated with atopic dermatitis, another chronic skin disease that is increasing in prevalence, affecting 10-20% of children and 1-3% of adults in industrial countries with an economic impact running into billions of dollars.

WO 2010/045584 A1 discloses conjugates which target siRNAs via the folate receptor to macrophages in order to treat inflammatory diseases.

Mati Fridkin et al. reports in Journal of Peptide Science Vol. 11, No. 1, 1 January 2005, pp. 37-44, to use a conjugate between Tuftsin and AZT for the treatment of AIDS.

Aouadi Myriam et al. discloses in Nature Vol. 458, No. 7242, p. 1180, the delivery of siRNA into macrophages for the suppression of systemic inflammation. Phagocytosis occurs via the dectin-1 receptor.

Ferkol, T. et al. discloses in PNAS, Vol. 93, 1 January 1996, pp. 101-105 the introduction of conjugates between mannosylated polylysine and genes into macrophages via the mannose receptor. D. Hristodorov et al. report in Toxins, molecular diversity preservation international (MDPI) AG, CH, about macrophague-targeted therapy: CD64-based immuno toxins for treatment of chronic inflammatory diseases.

### Brief description of the invention

One object of the invention is to provide an effective medicament for treating chronic inflammatory diseases.

Another object of the present invention is to provide a compound, which is able to treat chronic inflammatory diseases.

Targeting M1 macrophages during chronic inflammation could therefore be a promising intervention strategy to correct the imbalance between M1 and M2 macrophages and the resulting accumulation of pro-inflammatory cytokines thereby promoting the successful resolution of chronic inflammation.

The present invention is exemplified by the specific elimination of both murine and human M1 macrophages *in vitro* and *in vivo.* Notably, the elimination of M1 macrophages *in vivo* in a transgenic mouse model and *ex vivo* in a skin explant from a patient with atopic dermatitis changed the microenvironmental conditions in such a way as to promote the accumulation of anti-inflammatory M2 macrophages. In addition to M1-specific elimination, it is disclosed herein that both populations show phenotypic plasticity upon exposure to inverse stimuli (IL-4 for M1 and IFN-γ for M2). Repolarization proved to be possible in both directions (M1 to M2 and vice versa) in terms of the surface markers and the soluble cytokines, coinciding with the change in sensitivity towards H22(scFv)-ETA'.

The present invention uses an immunomodulation approach rather than cell killing to address chronic inflammatory diseases. A polarizing approach would not only affect tissue-infiltrating monocytes, but also tissue macrophages that are already polarized. Using CD64 as a gateway example, the present invention demonstrates exemplarily the potential of H22(scFv)-C/EBPβ (CCAAT-enhancer binding protein β) to switch pro-inflammatory M1 macrophages to an anti-inflammatory M2-like phenotype.

Together, targeting appropriate surface receptors of M1 macrophages using immunomodulatory fusion molecules will facilitate the development of novel intervention strategies for most chronic inflammatory diseases.

The object of the invention has been accomplished by a human immunomodulatory fusion protein (IFP) for converting pro-inflammatory M1 macrophages to anti-inflammatory M2 like phenotypes of macrophages the IFP having at least one component A and at least one component B wherein the component A is comprising or consisting of a binding domain for extra-cellular surface structures of a macrophage that internalizes said immunomodulatory fusion protein (IFP)upon binding of component A of said immunomodulatory fusion protein (IFP), and the component B is murine C/EBPβ, or human C/EBPβ.

The immunomodulatory fusion protein (IFP)of the invention is a human immuno modulatory fusion protein (IFP).

In another embodiment of the invention the component A of the immunomodulatory fusion protein (IFP) of the invention can be selected from the group of internalizing macrophage-specific cell surface receptor binding structures comprising or consisting of antibodies or their derivatives or fragments thereof, synthetic peptides such as scFv, mimotopes, etc. or chemical molecules such as carbohydrates, lipids, nucleic acids, peptides, vitamins, etc., and/or small molecules with up to 100 atoms with receptor-binding activity like ligands, in particular peptidic molecules, non-peptidic molecules, etc., and/or carbohydrate binding proteins and their ligands such as lectins, in particular calnexins, c-type lectins, I-type lectins, m-type lectins, p-type lectins, r-type lectins, galectins and their derivatives, and/or receptor binding molecules such as natural ligands to macrophage-specific cluster of differentiation (CD) antigens, like CD11c, CD14, CD64, etc., cytokines such as chemokines, colony stimulating factors, type-1 cytokines, type-2 cytokines, interferons, interleukins, lymphokines, monokines, etc., and/or adhesion molecules including their derivatives and mu tants, and/or derivatives or combinations of any of the above listed binding structures.

In yet another embodiment, component A of the immunomodulatory fusion protein (IFP) of the invention may be selected from the following list:
CD64, CD11c, CD14, CD16b, CD25, CD36, CD39, CD80, CD86, CD89, CD273, CD284, HLA-A major histocompatibility complex, class I, A, HLA-B major histocompatibility complex, class I, B, HLA-C major histocompatibility complex, class I, C, HLA-E major histocompatibility complex, class I, E, HLA-F major histocompatibility complex, class I, F, HLA-G major histocompatibility complex, class I, G, HLA-DQA1 major histocompatibility complex, class II, DQ alpha 1, HLA-DQA2 major histocompatibility complex, class II, DQ alpha 2, HLA-DQB1 major histocompatibility complex, class II, DQ beta 1, HLA-DQB2 major histocompatibility complex, class II, DQ beta 2, HLA-DRA major histocompatibility complex, class II, DR alpha HLA-DRB1 major histocompatibility complex, class II, DR beta 1, HLA-DRB3 major histocompatibility complex, class II, DR beta 3, HLA-DRB4 major histocompatibility complex, class II, DR beta 4, HLA-DRB5 major histocompatibility complex, class II, DR beta 5, CD74 molecule, major histocompatibility complex, class II invariant chain, HLA-DPA1 major histocompatibility complex, class II, DP alpha 1, HLA-DPB1 major histocompatibility complex, class II, DP beta 1, HLA-DMA major histocompatibility complex, class II, DM alpha, HLA-DMB major histocompatibility complex, class II, DM beta, HLA-DOA major histocompatibility complex, class II, DO alpha, HLA-DOB major histocompatibility complex, class II, DO beta.

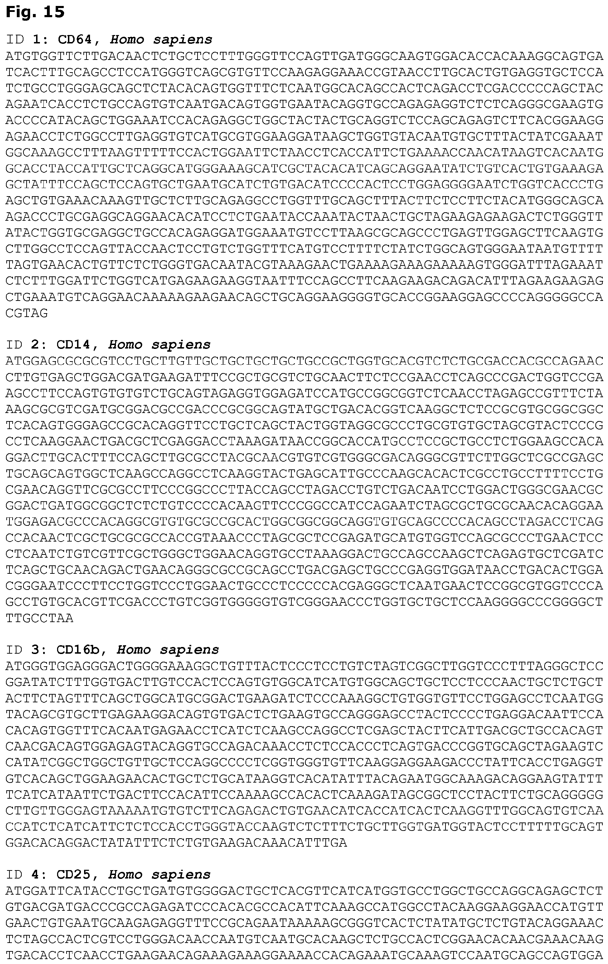

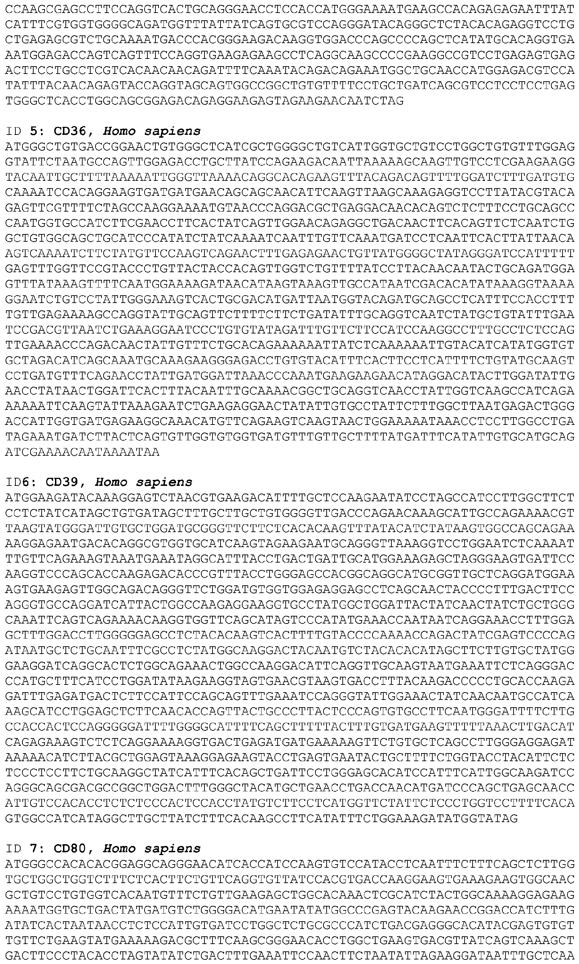

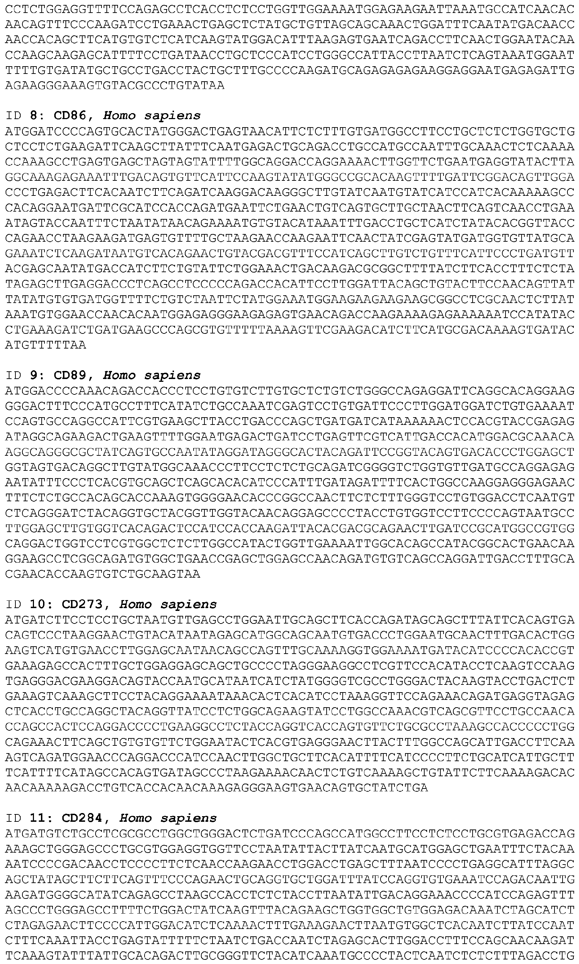

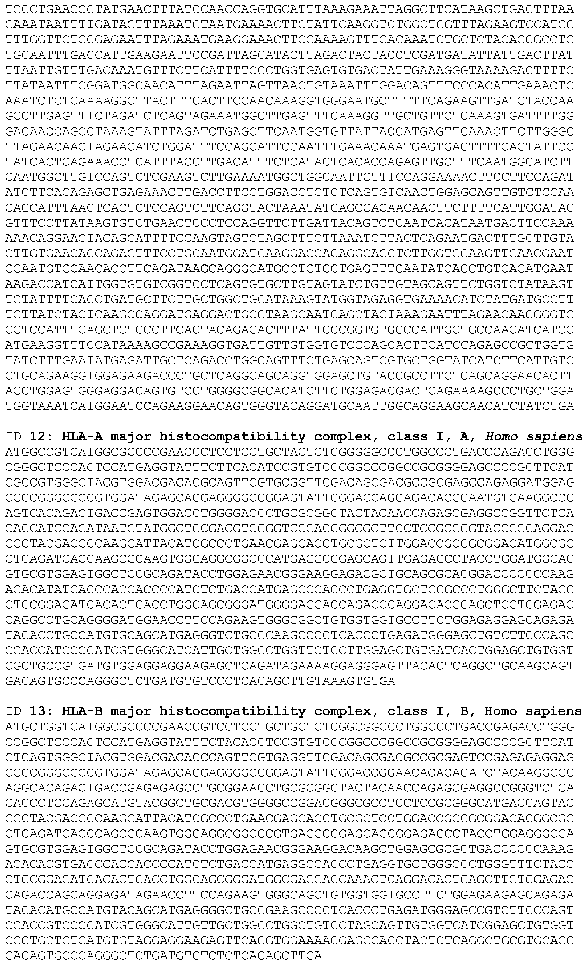

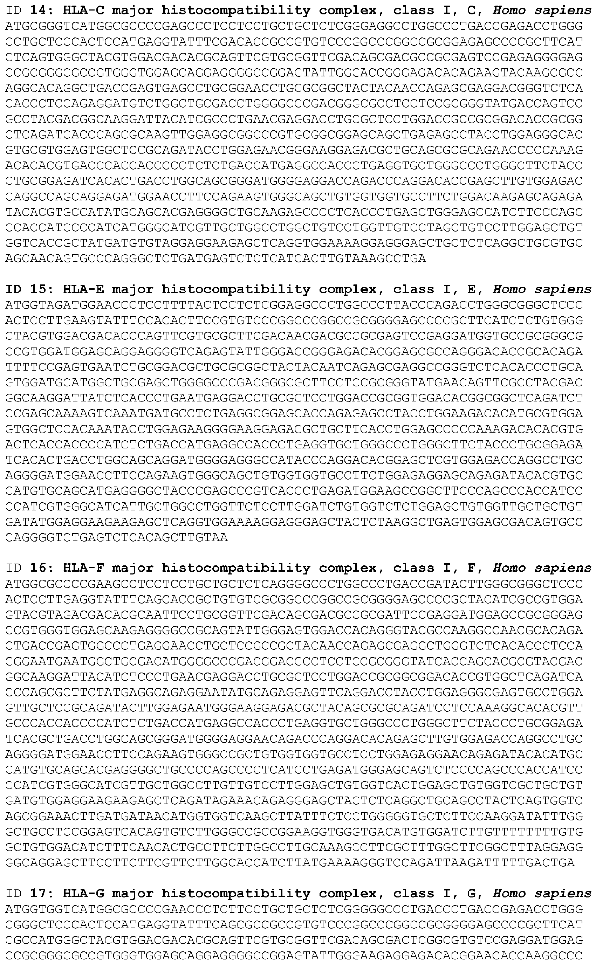

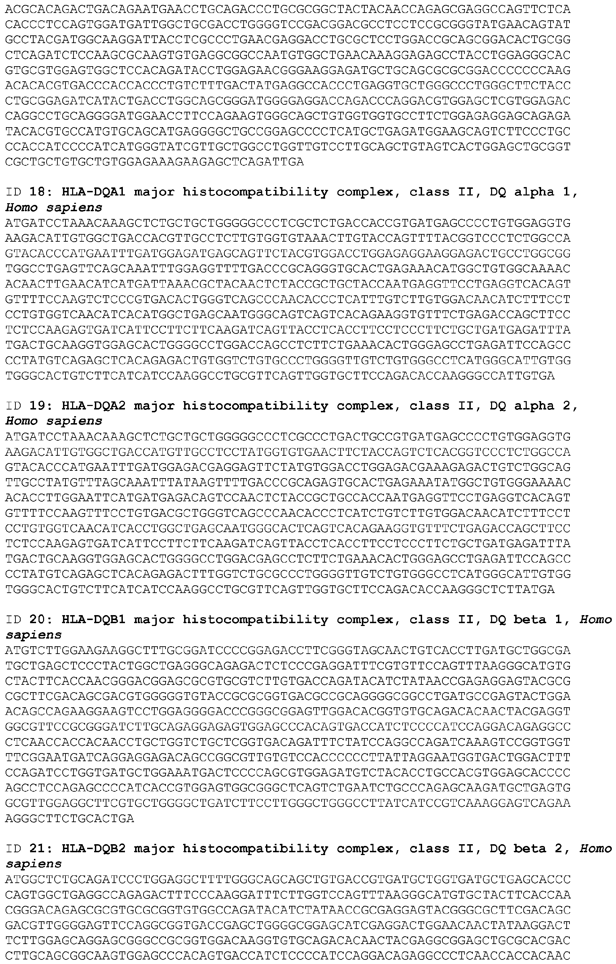

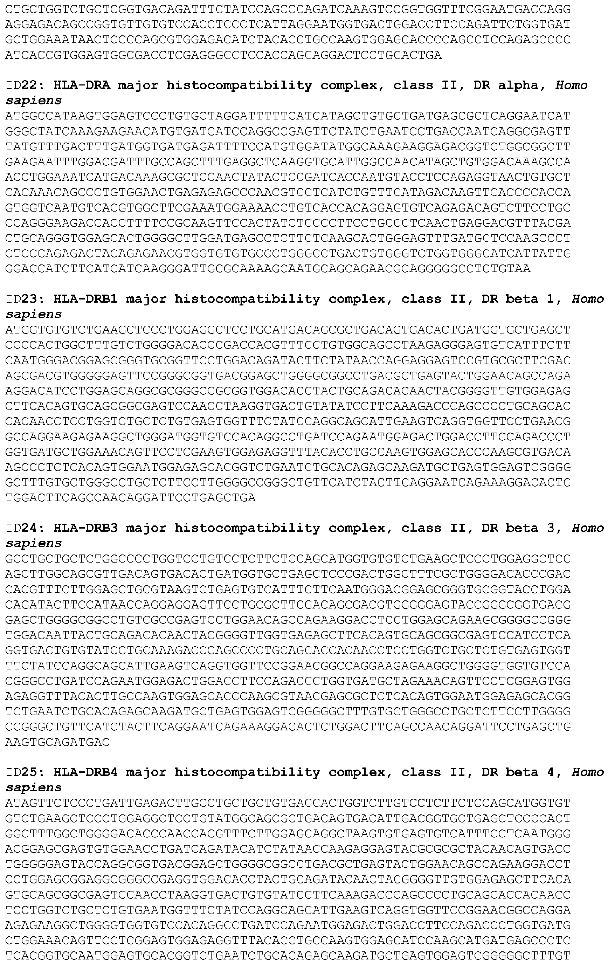

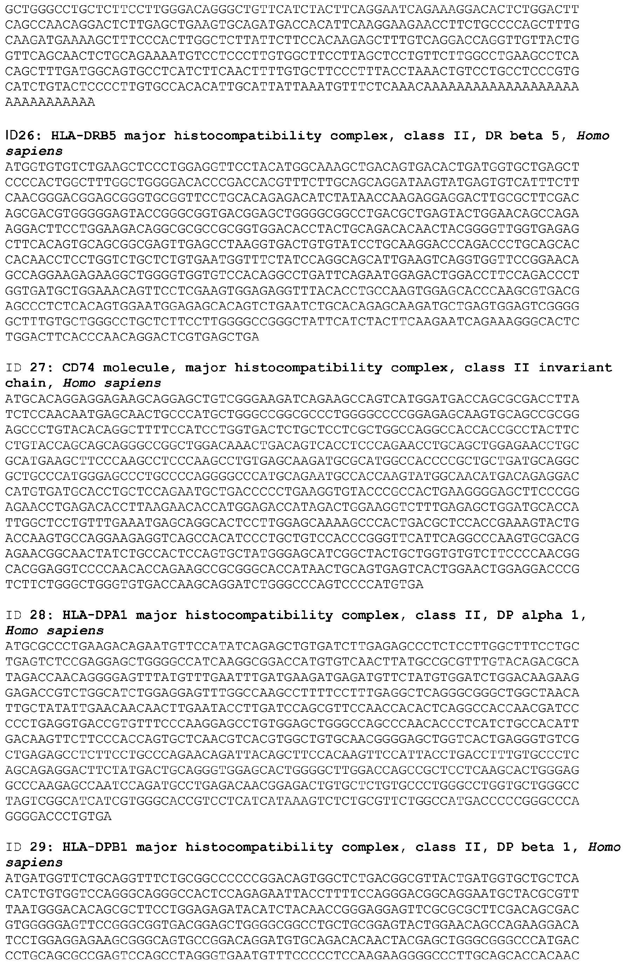

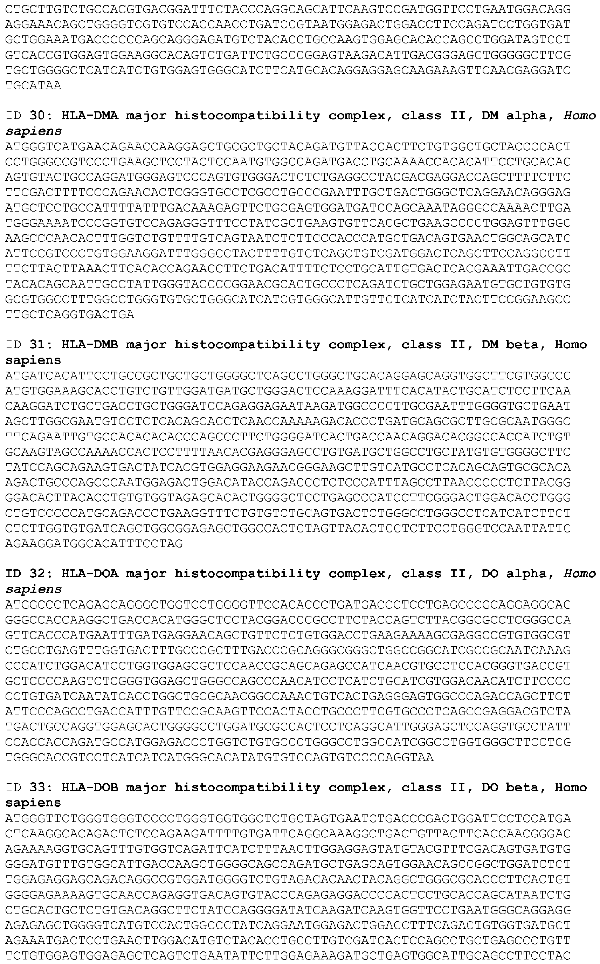

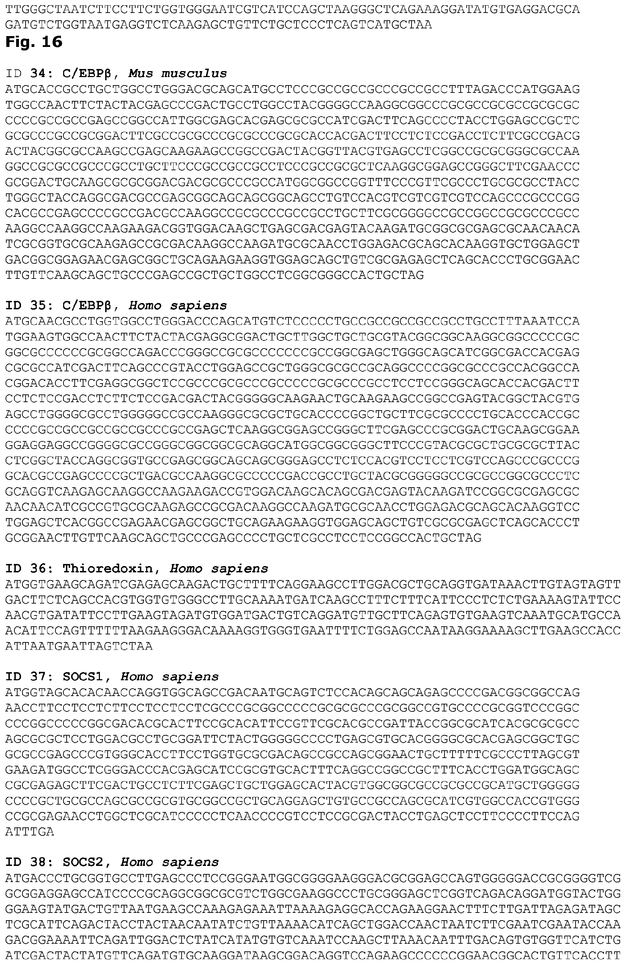

In particular, component A of the immunomodulatory fusion protein (IFP) of the invention may specifically be selected from the polypeptides of Seq ID Nos. 1-33 (Fig. 15).

In a further embodiment of the invention the component A of the immunomodulatory fusion protein (IFP) of the invention can be a chemokine or a specifically binding fragment thereof.

In still a further embodiment of the invention the component A of the immunomodulatory fusion protein (IFP) of the invention may be selected from the group consisting of CXC chemokine/receptor family such as CXCL1, CXCL2, CXCL3, CXCL4, CXCL5, CXCL6, CXCL7, CXCL8, CXCL9, CXCL10, CXCL11, CXCL12, CXCL13, CXCL14, KXCL15, CXCL16; or the C chemokine /receptor family, such as XCL1 and XCL2; or the CX₃C chemokine/receptor family, such as CX3CL1, or the CC chemokine/receptor family, such as CCL1, CCL2, CCL3, CCL3L1, CCL4, CCLS, (CCL6), CCL7, CCL8, (CCL9/10), CCL11, (CCL12)CCL13, CCL14, CCL15, CCL16, CCL17, CCL18, CCL19, CCL20, CCL21, CCL22, CCL23, CCL24, CCL25, CCL26, CCL27, and CCL28.

In yet a further embodiment of the invention the component A of the immunomodulatory fusion protein (IFP) of the invention can be an interleukin or a specifically binding fragment thereof like those provided in table 2 binding to its specific cellular receptor. The interleukins are in particular selected from the group consisting of IL-1; IL-2; IL-3; IL-4; IL-5; IL-6; IL-7; IL-8; IL-9; IL-10; IL-11; IL-12; IL-13; IL-14; IL-15; IL-16; IL-17; IL-18; IL-19; IL-20; IL-21; IL-22; IL-23; IL-24; IL-25; IL-26; IL-27; IL-28; IL-29; IL-30; IL-31; IL-32; IL-33; and IL-35.

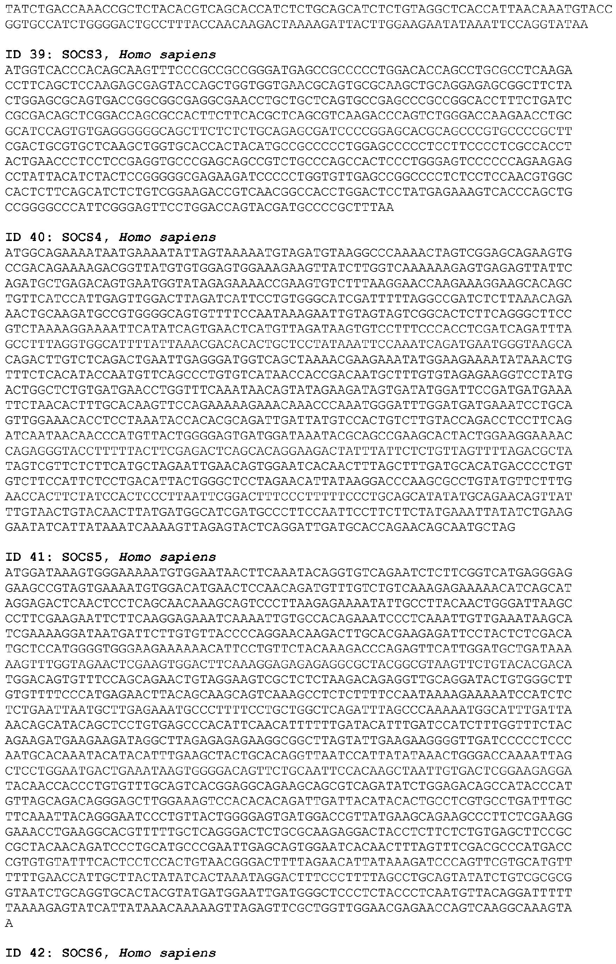

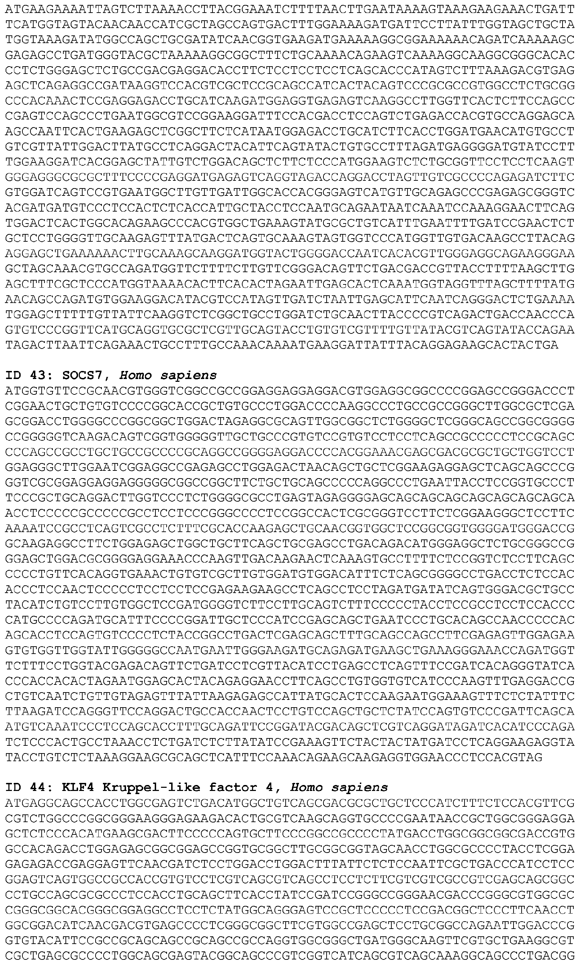

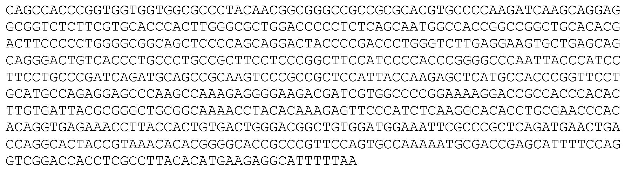

The component B of the immunomodulatory fusion protein (IFP) of the invention is selected from the group consisting of murine C/EBPβ, human C/EBPβ, such as exemplified by the proteins having one of the amino acid sequences encoded by the polynucleotide of one of the Seq ID Nos. 34 - 44 in Fig. 16.

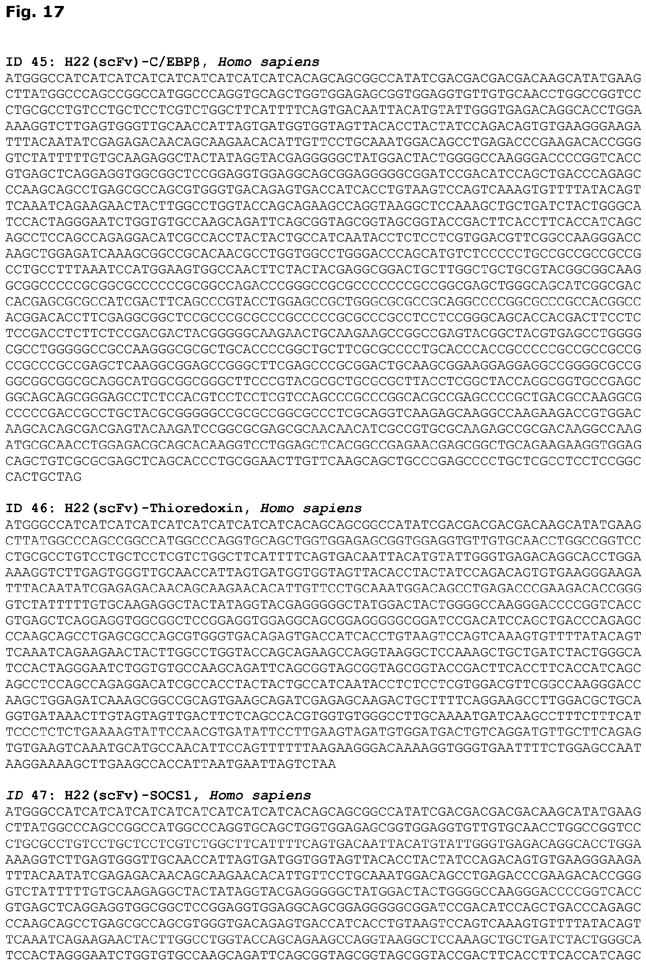

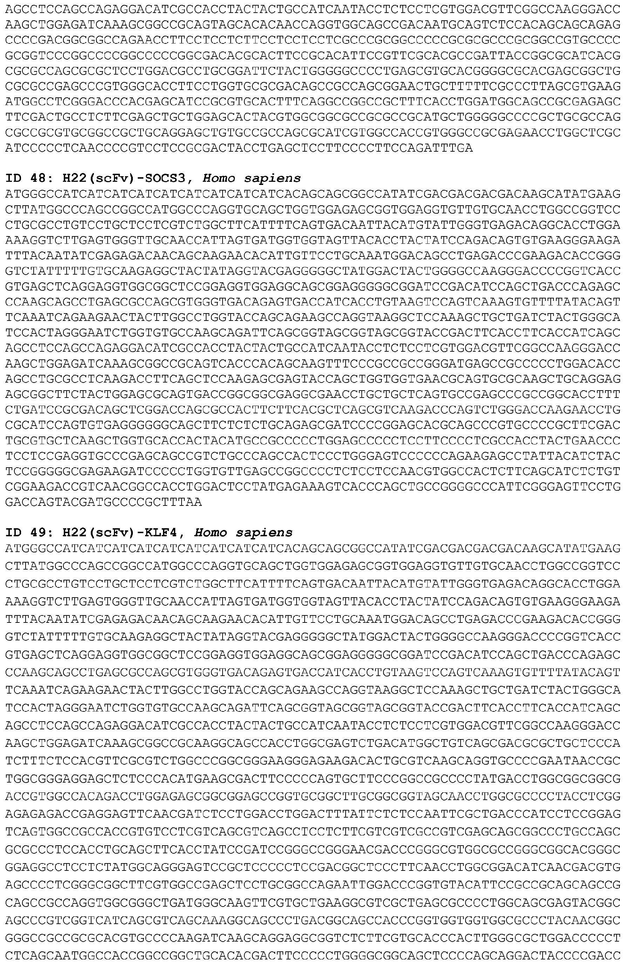

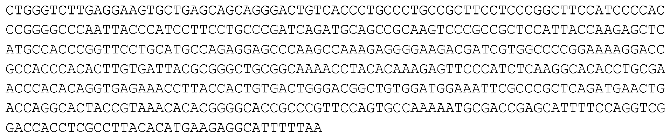

According to the invention the components A and B of the immunomodulatory fusion protein (IFP) of the invention are chemically coupled or fused to each other by genetic engineering, such as exemplified by the proteins having one of the amino acid sequences encoded by the polynucleotide of one of the Seq ID Nos. 45 - 49 (Fig. 17).

Subject matter of the present invention is also a process for manufacturing the immunomodulatory fusion protein (IFP) of the invention comprising or consisting of the steps
- cloning the component B to yield a polynucleotide; and
- fuse said polynucleotide coding for component B with a polynucleotide coding for a protein of component A to yield a polynucleotide coding for the immunomodulatory fusion protein (IFP) of the invention;
- expressing said polynucleotide coding for the immunomodulatory fusion protein (IFP) of the invention in suitable host, such as *E. coli;*
- isolation and purification of the immunomodulatory fusion protein (IFP) of the invention.

A further subject matter of the present invention is a polynucleotide encoding for the proteineous immunomodulatory fusion protein (IFP) or proteineous components of the immunomodulatory fusion protein (IFP) of the invention.

Another subject matter of the present invention is a vector comprising or consisting of at least one polynucleotide of the invention.

A further subject matter of the present invention is a cell having the vector of the invention.

A still further subject matter of the present invention is a medicament comprising or consisting of a immunomodulatory fusion protein (IFP) of the invention. Another subject matter of the invention is the immunomodulatory fusion protein (IFP) of the invention which can be used in the treatment of chronic inflammatory diseases.

The immunomodulatory fusion protein of the invention is in particular suitable for use in converting pro-inflammatory M1 macrophages in anti-inflammarory M2-like phenotype in the treatment of chronic inflammatory diseases.

Consequently the immunomodulatory fusion protein (IFP) of the invention can be used in a method of converting pro-inflammatory M1 macrophage in an anti-inflammarory M2-like phenotype by contacting the M1 macrophage with the immunomodulatory fusion protein of the invention for a sufficient time to effect the conversion of the M1 macrophage to the anti-inflammarory M2-like phenotype.

Likewise the immunomodulatory fusion protein (IFP) of the invention can be used in a method of treating an inflammation by converting pro-inflammatory M1 macrophage in an anti-inflammarory M2-like phenotype by contacting the M1 macrophage with the immunomodulatory fusion protein of the invention for a sufficient time to effect the conversion of the M1 macrophage to the anti-inflammarory M2-like phenotype.

### Brief description of the figures

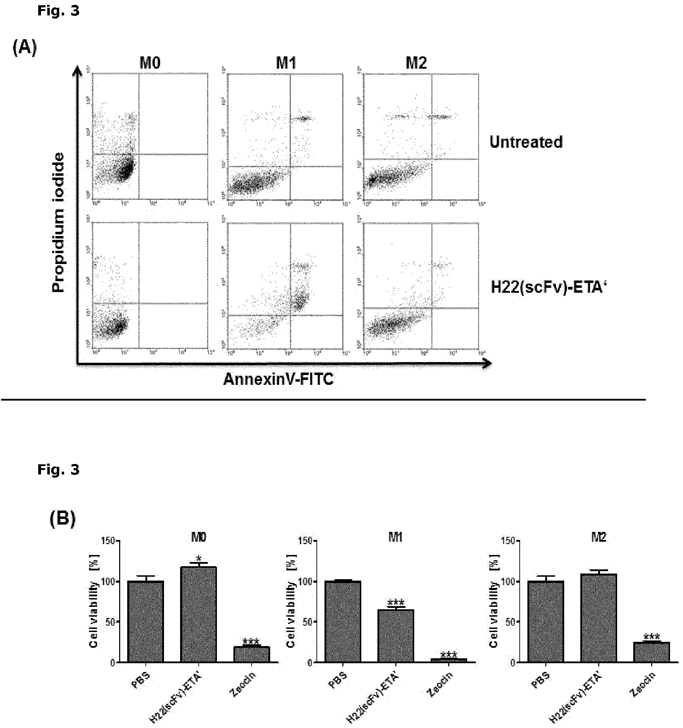

**Fig. 1**
   a: Shows a literature-based tentative hypothesis for the initiation and amplification of an inflammatory response.
   b: Shows screening of M1-specific and M2-specific surface markers on hCD64tg murine macrophages. M1/M2 ratio > 0 = upregulation in M1 cells; M1/M2 ratio < 0 = upregulation in M2 cells.
   c: Shows screening of M1-specific and M2-specific surface markers on human PBMC-derived macrophages. M1/M2 ratio > 0 = upregulation in M1 cells; M1/M2 ratio < 0 = upregulation in M2 cells.
   d: Shows the expression of soluble cytokines by polarized macrophages. Values are means + SD; ***p≤0.001 (unpaired two-tailed Student's *t*-test).

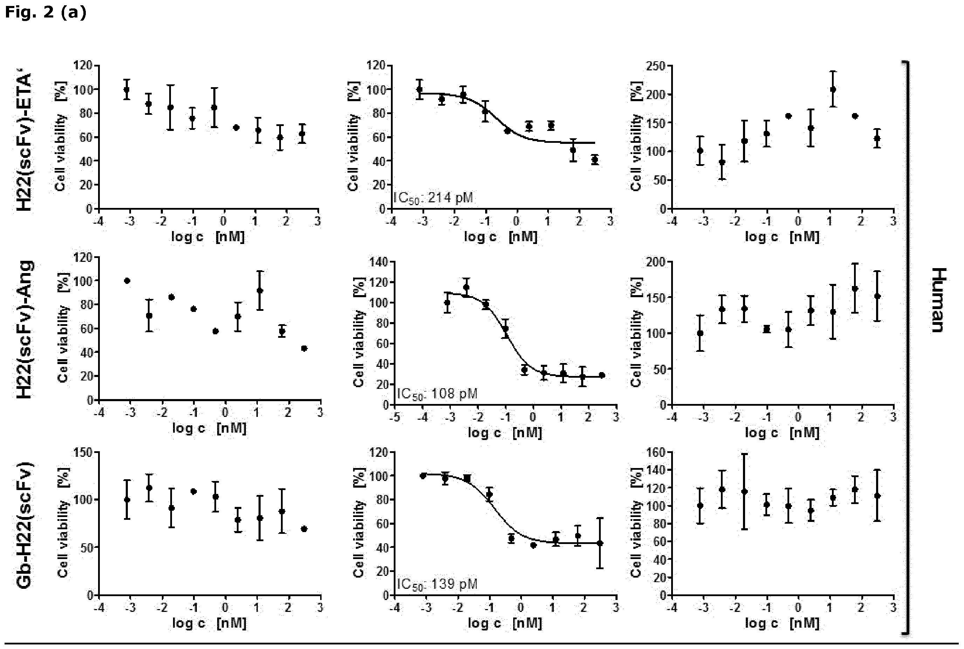

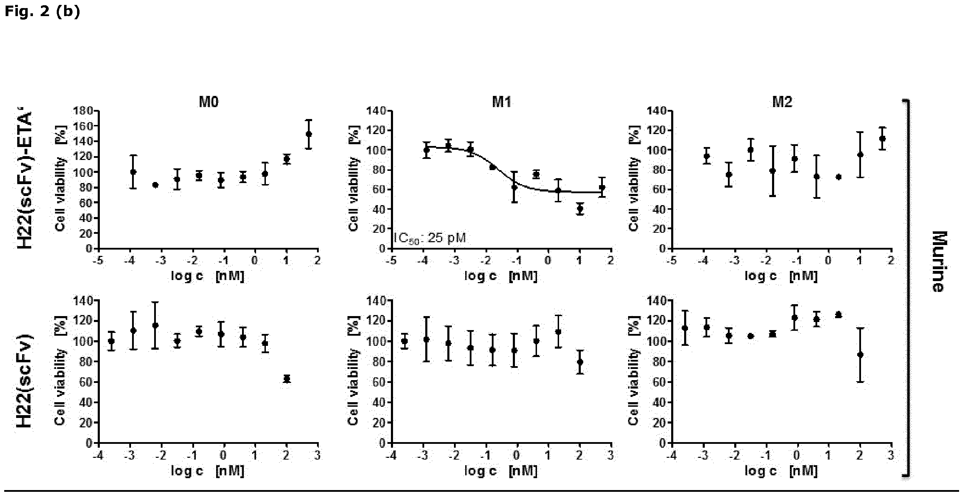

**Fig. 2****:** Shows M1-specific elimination of polarized macrophages *in vitro.*
**Fig. 3**
   a: Shows the selective induction of apoptosis in M1 macrophages by H22(scFv)-ETA'.
   b: Shows the quantitative analysis of the results obtained in Fig. 3a. Zeocin was used as a control. Values are means + SD; *p≤0.05, ***p≤0.001 (one-way ANOVA).
**Fig. 4**
   a: Shows the selective elimination of M1 macrophages in transgenic mice *in vivo.*
   b: Shows representative images of murine skin sections stained for M1-specific and M2-specific markers. Examples of stained cells are indicated by arrows. Objective: 10x.
   c: Shows that the selective elimination of M1 macrophages shifts the M1/M2 ratio to an anti-inflammatory state in mouse skin.
**Fig. 5**
   a: Shows an H&E staining of untreated skin demonstrating the infiltration of inflammatory cells.
   b: Shows representative images of human skin sections stained for M1-specific and M2-specific markers after *ex vivo* treatment with H22(scFv)-ETA'. Examples of stained cells are indicated by arrows. Objective: 10x.
   c: Shows that treatment with H22(scFv)-ETA' reduced the number of CD64⁺CD14⁺ M1 cells compared with the control incubated in medium only. In contrast, the number of CD206⁺CD301⁺ M2 macrophages increased. Values are means + SD; *p≤0.05, **p:50.01 (one-way ANOVA).
   d: Shows that the selective elimination of M1 macrophages shifts the M1/M2 ratio to an anti-inflammatory state in human skin.
   e: Shows that the elimination of M1 macrophages reduced the level of the pro-inflammatory IL-6 and induced the production of the anti-inflammatory IL-10. Values are means + SD; ***p≤0.001 (one-way ANOVA).
**Fig. 6**
   a: Shows phenotypic plasticity of polarized macrophages *in vitro* (surface markers).
   b: Shows phenotypic plasticity of polarized macrophages *in vitro* (soluble markers). Values are means + SD; ***p≤0.001 (one-way ANOVA). MC, medium change.
   c: Shows functional plasticity of polarized macrophages *in vitro.*
**Fig. 7****:** Shows the vector map of H22(scFv)-C/EBPβ.
**Fig. 8****:** Shows the expression cassette of H22(scFv)-C/EBPβ.
**Fig. 9**
   a: Shows a Coomassie Brilliant Blue stained SDS-PAGE gel of H22(scFv)-C/EBPβ.
   b: Shows a western blot of H22(scFv)-C/EBPβ.

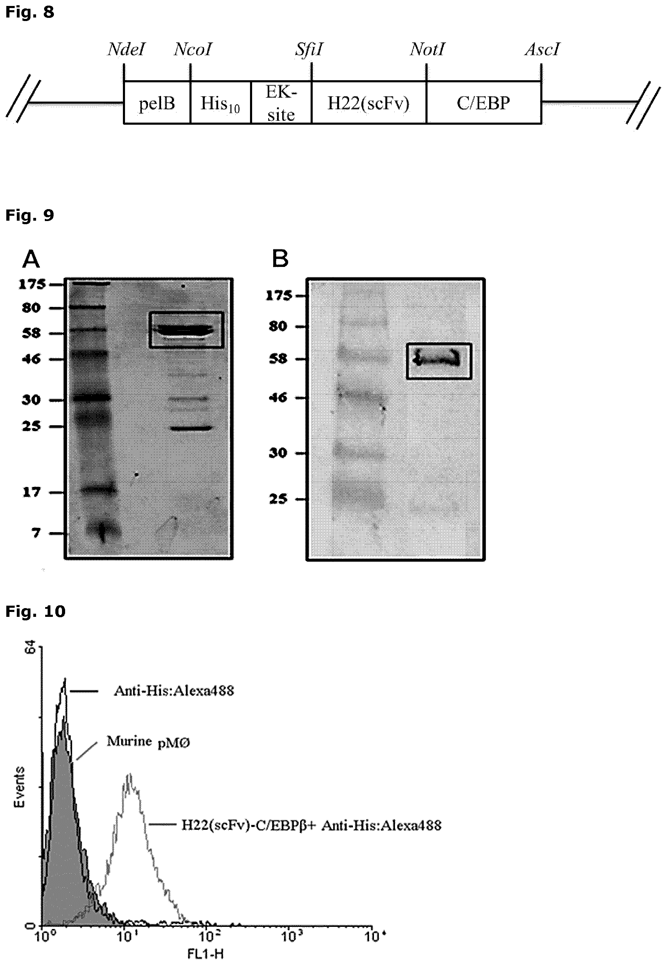

**Fig. 10****:** Shows binding of H22(scFv)-C/EBPβ to target cells.

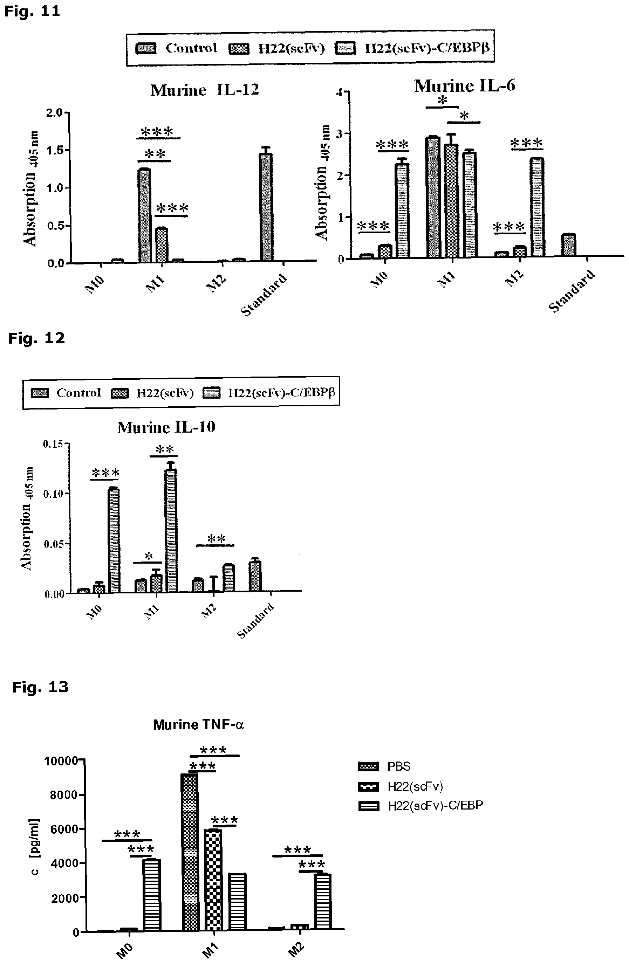

**Fig. 11****:** Shows the reduction of pro-inflammatory cytokines (IL-12 and IL-6) in M1 macrophages induced by H22(scFv)-C/EBPβ.
**Fig. 12****:** Shows the induction of anti-inflammatory cytokines in M1 macrophages by H22(scFv)-C/EBPβ.
**Fig. 13****:** Shows the reduction of the pro-inflammatory cytokine TNF-α in M1 macrophages induced by H22(scFv)-C/EBPβ.

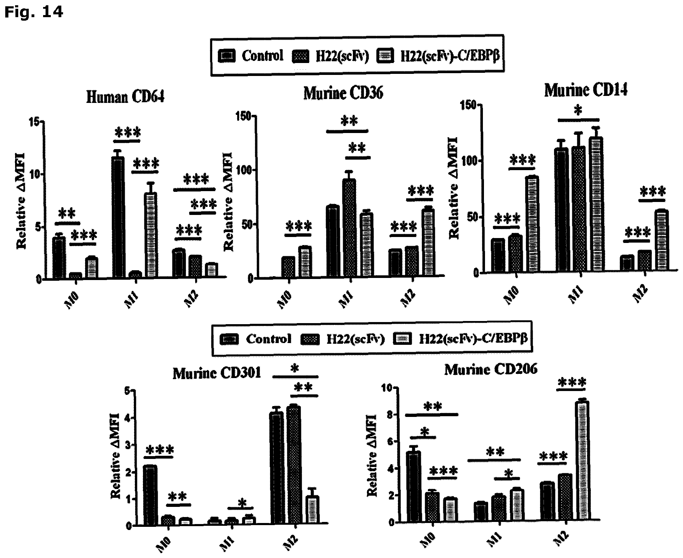

**Fig. 14****:** Shows the impact of H22(scFv)-C/EBPβ on the expression of surface markers and the induction of a hybrid phenotype (CD14^{high}/cD36^{high}/cD206^{high}/cD301^{low}) in M2 macrophages.
**Fig. 15****:** Shows ORFs of exemplified surface targets expressed by M1 macrophages.
**Fig. 16****:** Shows ORFs of exemplified macrophage modulatory proteins.
**Fig. 17****:** Shows ORFs of exemplified immunomodulatory fusion proteins.

### Detailed description of the invention

The present invention pertains to a human immunomodulatory fusion protein (IFP) formed from at least one component A comprising or consisting of a binding domain for extra-cellular surface structures of macrophages that internalizes upon binding of component A of said immunomodulatory fusion protein (IFP), and at least one component B, characterized in that component B is murine or human C/EBPβ.

In one embodiment of the invention the component A of the immunomodulatory fusion protein (IFP) of the invention is selected from the group of internalizing cell surface receptor binding structures consisting of antibodies or their derivatives or fragments thereof, synthetic peptides such as scFv, mimotopes, etc. or chemical molecules such as carbohydrates, lipids, nucleic acids, peptides, vitamins, etc., and/or small molecules with up to 100 atoms with receptor-binding activity like ligands, in particular single atoms, peptidic molecules, non-peptidic molecules, etc., and/or carbohydrate binding proteins and their ligands such as lectins, in particular calnexins, c-type lectins, I-type lectins, m-type lectins, p-type lectins, r-type lectins, galectins and their derivatives, and/or receptor binding molecules such as natural ligands to the macropage-specific cluster of differentiation (CD) antigens, like CD11c, CD14, CD64, etc., cytokines such as chemokines, colony stimulating factors, type-1 cytokines, type-2 cytokines, interferons, interleukins, lymphokines, monokines, etc., and/or adhesion molecules including their derivatives and mutants, and/or derivatives or combinations of any of the above listed binding structures.

Component A is in particular binding to a cell surface marker of macrophages belonging to the cluster of differentiation antigens and major histocompatibility complex antigens. For example, component A comprises or consists of a immunomodulatory fusion protein (IFP) selected from the group consisting of CD64, CD11c, CD14, CD16b, CD25, CD36, CD39, CD80, CD86, CD89, CD273, CD284, HLA-A major histocompatibility complex, class I, A, HLA-B major histocompatibility complex, class I, B, HLA-C major histocompatibility complex, class I, C, HLA-E major histocompatibility complex, class I, E, HLA-F major histocompatibility complex, class I, F, HLA-G major histocompatibility complex, class I, G, HLA-DQA1 major histocompatibility complex, class II, DQ alpha 1, HLA-DQA2 major histocompatibility complex, class II, DQ alpha 2, HLA-DQB1 major histocompatibility complex, class II, DQ beta 1, HLA-DQB2 major histocompatibility complex, class II, DQ beta 2, HLA-DRA major histocompatibility complex, class II, DR alpha HLA-DRB1 major histocompatibility complex, class II, DR beta 1, HLA-DRB3 major histocompatibility complex, class II, DR beta 3, HLA-DRB4 major histocompatibility complex, class II, DR beta 4, HLA-DRB5 major histocompatibility complex, class II, DR beta 5, CD74 molecule, major histocompatibility complex, class II invariant chain, HLA-DPA1 major histocompatibility complex, class II, DP alpha 1, HLA-DPB1 major histocompatibility complex, class II, DP beta 1, HLA-DMA major histocompatibility complex, class II, DM alpha, HLA-DMB major histocompatibility complex, class II, DM beta, HLA-DOA major histocompatibility complex, class II, DO alpha, HLA-DOB major histocompatibility complex, class II, and DO beta which may be coupled with the cytotoxic component B.

The immunomodulatory fusion protein (IFP) of the invention may comprise a compound selected from the group consisting of CD64, CD14, CD16b, CD25, CD36, CD39, CD80, CD86, CD89, CD273, CD284, HLA-A major histocompatibility complex, class I, A, HLA-B major histocompatibility complex, class I, B, HLA-C major histocompatibility complex, class I, C, HLA-E major histocompatibility complex, class I, E, HLA-F major histocompatibility complex, class I, F, HLA-G major histocompatibility complex, class I, G, HLA-DQA1 major histocompatibility complex, class II, DQ alpha 1, HLA-DQA2 major histocompatibility complex, class II, DQ alpha 2, HLA-DQB1 major histocompatibility complex, class II, DQ beta 1, HLA-DQB2 major histocompatibility complex, class II, DQ beta 2, HLA-DRA major histocompatibility complex, class II, DR alpha HLA-DRB1 major histocompatibility complex, class II, DR beta 1, HLA-DRB3 major histocompatibility complex, class II, DR beta 3, HLA-DRB4 major histocompatibility complex, class II, DR beta 4, HLA-DRB5 major histocompatibility complex, class II, DR beta 5, CD74 molecule, major histocompatibility complex, class II invariant chain, HLA-DPA1 major histocompatibility complex, class II, DP alpha 1, HLA-DPB1 major histocompatibility complex, class II, DP beta 1, HLA-DMA major histocompatibility complex, class II, DM alpha, HLA-DMB major histocompatibility complex, class II, DM beta, HLA-DOA major histocompatibility complex, class II, DO alpha, HLA-DOB major histocompatibility complex, class II, and DO beta and is coupled with a macrophage modulatory protein.

The immunomodulatory fusion protein (IFP) of the invention may further comprise a compound selected from the group consisting of CD64, CD14, CD16b, CD25, CD36, CD39, CD80, CD86, CD89, CD273, CD284, HLA-A major histocompatibility complex, class I, A, HLA-B major histocompatibility complex, class I, B, HLA-C major histocompatibility complex, class I, C, HLA-E major histocompatibility complex, class I, E, HLA-F major histocompatibility complex, class I, F, HLA-G major histocompatibility complex, class I, G, HLA-DQA1 major histocompatibility complex, class II, DQ alpha 1, HLA-DQA2 major histocompatibility complex, class II, DQ alpha 2, HLA-DQB1 major histocompatibility complex, class II, DQ beta 1, HLA-DQB2 major histocompatibility complex, class II, DQ beta 2, HLA-DRA major histocompatibility complex, class II, DR alpha HLA-DRB1 major histocompatibility complex, class II, DR beta 1, HLA-DRB3 major histocompatibility complex, class II, DR beta 3, HLA-DRB4 major histocompatibility complex, class II, DR beta 4, HLA-DRB5 major histocompatibility complex, class II, DR beta 5, CD74 molecule, major histocompatibility complex, class II invariant chain, HLA-DPA1 major histocompatibility complex, class II, DP alpha 1, HLA-DPB1 major histocompatibility complex, class II, DP beta 1, HLA-DMA major histocompatibility complex, class II, DM alpha, HLA-DMB major histocompatibility complex, class II, DM beta, HLA-DOA major histocompatibility complex, class II, DO alpha, HLA-DOB major histocompatibility complex, class II, and DO beta and is coupled with murine C/EBPβ.

The immunomodulatory fusion protein (IFP) of the invention may further comprise a compound selected from the group consisting of CD64, CD14, CD16b, CD25, CD36, CD39, CD80, CD86, CD89, CD273, CD284, HLA-A major histocompatibility complex, class I, A, HLA-B major histocompatibility complex, class I, B, HLA-C major histocompatibility complex, class I, C, HLA-E major histocompatibility complex, class I, E, HLA-F major histocompatibility complex, class I, F, HLA-G major histocompatibility complex, class I, G, HLA-DQA1 major histocompatibility complex, class II, DQ alpha 1, HLA-DQA2 major histocompatibility complex, class II, DQ alpha 2, HLA-DQB1 major histocompatibility complex, class II, DQ beta 1, HLA-DQB2 major histocompatibility complex, class II, DQ beta 2, HLA-DRA major histocompatibility complex, class II, DR alpha HLA-DRB1 major histocompatibility complex, class II, DR beta 1, HLA-DRB3 major histocompatibility complex, class II, DR beta 3, HLA-DRB4 major histocompatibility complex, class II, DR beta 4, HLA-DRB5 major histocompatibility complex, class II, DR beta 5, CD74 molecule, major histocompatibility complex, class II invariant chain, HLA-DPA1 major histocompatibility complex, class II, DP alpha 1, HLA-DPB1 major histocompatibility complex, class II, DP beta 1, HLA-DMA major histocompatibility complex, class II, DM alpha, HLA-DMB major histocompatibility complex, class II, DM beta, HLA-DOA major histocompatibility complex, class II, DO alpha, HLA-DOB major histocompatibility complex, class II, and DO beta and is coupled with human C/EBPβ.

The immunomodulatory fusion protein (IFP) of the invention may further comprise a compound selected from the group consisting of CD64, CD14, CD16b, CD25, CD36, CD39, CD80, CD86, CD89, CD273, CD284, HLA-A major histocompatibility complex, class I, A, HLA-B major histocompatibility complex, class I, B, HLA-C major histocompatibility complex, class I, C, HLA-E major histocompatibility complex, class I, E, HLA-F major histocompatibility complex, class I, F, HLA-G major histocompatibility complex, class I, G, HLA-DQA1 major histocompatibility complex, class II, DQ alpha 1, HLA-DQA2 major histocompatibility complex, class II, DQ alpha 2, HLA-DQB1 major histocompatibility complex, class II, DQ beta 1, HLA-DQB2 major histocompatibility complex, class II, DQ beta 2, HLA-DRA major histocompatibility complex, class II, DR alpha HLA-DRB1 major histocompatibility complex, class II, DR beta 1, HLA-DRB3 major histocompatibility complex, class II, DR beta 3, HLA-DRB4 major histocompatibility complex, class II, DR beta 4, HLA-DRB5 major histocompatibility complex, class II, DR beta 5, CD74 molecule, major histocompatibility complex, class II invariant chain, HLA-DPA1 major histocompatibility complex, class II, DP alpha 1, HLA-DPB1 major histocompatibility complex, class II, DP beta 1, HLA-DMA major histocompatibility complex, class II, DM alpha, HLA-DMB major histocompatibility complex, class II, DM beta, HLA-DOA major histocompatibility complex, class II, DO alpha, HLA-DOB major histocompatibility complex, class II, and DO beta and is coupled with Thioredoxin.

The immunomodulatory fusion protein (IFP) of the invention may further comprise a compound selected from the group consisting of CD64, CD14, CD16b, CD25, CD36, CD39, CD80, CD86, CD89, CD273, CD284, HLA-A major histocompatibility complex, class I, A, HLA-B major histocompatibility complex, class I, B, HLA-C major histocompatibility complex, class I, C, HLA-E major histocompatibility complex, class I, E, HLA-F major histocompatibility complex, class I, F, HLA-G major histocompatibility complex, class I, G, HLA-DQA1 major histocompatibility complex, class II, DQ alpha 1, HLA-DQA2 major histocompatibility complex, class II, DQ alpha 2, HLA-DQB1 major histocompatibility complex, class II, DQ beta 1, HLA-DQB2 major histocompatibility complex, class II, DQ beta 2, HLA-DRA major histocompatibility complex, class II, DR alpha HLA-DRB1 major histocompatibility complex, class II, DR beta 1, HLA-DRB3 major histocompatibility complex, class II, DR beta 3, HLA-DRB4 major histocompatibility complex, class II, DR beta 4, HLA-DRB5 major histocompatibility complex, class II, DR beta 5, CD74 molecule, major histocompatibility complex, class II invariant chain, HLA-DPA1 major histocompatibility complex, class II, DP alpha 1, HLA-DPB1 major histocompatibility complex, class II, DP beta 1, HLA-DMA major histocompatibility complex, class II, DM alpha, HLA-DMB major histocompatibility complex, class II, DM beta, HLA-DOA major histocompatibility complex, class II, DO alpha, HLA-DOB major histocompatibility complex, class II, and DO beta and is coupled with SOCS1.

The immunomodulatory fusion protein (IFP) of the invention may further comprise a compound selected from the group consisting of CD64, CD14, CD16b, CD25, CD36, CD39, CD80, CD86, CD89, CD273, CD284, HLA-A major histocompatibility complex, class I, A, HLA-B major histocompatibility complex, class I, B, HLA-C major histocompatibility complex, class I, C, HLA-E major histocompatibility complex, class I, E, HLA-F major histocompatibility complex, class I, F, HLA-G major histocompatibility complex, class I, G, HLA-DQA1 major histocompatibility complex, class II, DQ alpha 1, HLA-DQA2 major histocompatibility complex, class II, DQ alpha 2, HLA-DQB1 major histocompatibility complex, class II, DQ beta 1, HLA-DQB2 major histocompatibility complex, class II, DQ beta 2, HLA-DRA major histocompatibility complex, class II, DR alpha HLA-DRB1 major histocompatibility complex, class II, DR beta 1, HLA-DRB3 major histocompatibility complex, class II, DR beta 3, HLA-DRB4 major histocompatibility complex, class II, DR beta 4, HLA-DRB5 major histocompatibility complex, class II, DR beta 5, CD74 molecule, major histocompatibility complex, class II invariant chain, HLA-DPA1 major histocompatibility complex, class II, DP alpha 1, HLA-DPB1 major histocompatibility complex, class II, DP beta 1, HLA-DMA major histocompatibility complex, class II, DM alpha, HLA-DMB major histocompatibility complex, class II, DM beta, HLA-DOA major histocompatibility complex, class II, DO alpha, HLA-DOB major histocompatibility complex, class II, and DO beta and is coupled with SOCS2.

The immunomodulatory fusion protein (IFP) of the invention may further comprise a compound selected from the group consisting of CD64, CD14, CD16b, CD25, CD36, CD39, CD80, CD86, CD89, CD273, CD284, HLA-A major histocompatibility complex, class I, A, HLA-B major histocompatibility complex, class I, B, HLA-C major histocompatibility complex, class I, C, HLA-E major histocompatibility complex, class I, E, HLA-F major histocompatibility complex, class I, F, HLA-G major histocompatibility complex, class I, G, HLA-DQA1 major histocompatibility complex, class II, DQ alpha 1, HLA-DQA2 major histocompatibility complex, class II, DQ alpha 2, HLA-DQB1 major histocompatibility complex, class II, DQ beta 1, HLA-DQB2 major histocompatibility complex, class II, DQ beta 2, HLA-DRA major histocompatibility complex, class II, DR alpha HLA-DRB1 major histocompatibility complex, class II, DR beta 1, HLA-DRB3 major histocompatibility complex, class II, DR beta 3, HLA-DRB4 major histocompatibility complex, class II, DR beta 4, HLA-DRB5 major histocompatibility complex, class II, DR beta 5, CD74 molecule, major histocompatibility complex, class II invariant chain, HLA-DPA1 major histocompatibility complex, class II, DP alpha 1, HLA-DPB1 major histocompatibility complex, class II, DP beta 1, HLA-DMA major histocompatibility complex, class II, DM alpha, HLA-DMB major histocompatibility complex, class II, DM beta, HLA-DOA major histocompatibility complex, class II, DO alpha, HLA-DOB major histocompatibility complex, class II, and DO beta and is coupled with SOCS3.

The immunomodulatory fusion protein (IFP) of the invention may further comprise a compound selected from the group consisting of CD64, CD14, CD16b, CD25, CD36, CD39, CD80, CD86, CD89, CD273, CD284, HLA-A major histocompatibility complex, class I, A, HLA-B major histocompatibility complex, class I, B, HLA-C major histocompatibility complex, class I, C, HLA-E major histocompatibility complex, class I, E, HLA-F major histocompatibility complex, class I, F, HLA-G major histocompatibility complex, class I, G, HLA-DQA1 major histocompatibility complex, class II, DQ alpha 1, HLA-DQA2 major histocompatibility complex, class II, DQ alpha 2, HLA-DQB1 major histocompatibility complex, class II, DQ beta 1, HLA-DQB2 major histocompatibility complex, class II, DQ beta 2, HLA-DRA major histocompatibility complex, class II, DR alpha HLA-DRB1 major histocompatibility complex, class II, DR beta 1, HLA-DRB3 major histocompatibility complex, class II, DR beta 3, HLA-DRB4 major histocompatibility complex, class II, DR beta 4, HLA-DRB5 major histocompatibility complex, class II, DR beta 5, CD74 molecule, major histocompatibility complex, class II invariant chain, HLA-DPA1 major histocompatibility complex, class II, DP alpha 1, HLA-DPB1 major histocompatibility complex, class II, DP beta 1, HLA-DMA major histocompatibility complex, class II, DM alpha, HLA-DMB major histocompatibility complex, class II, DM beta, HLA-DOA major histocompatibility complex, class II, DO alpha, HLA-DOB major histocompatibility complex, class II, and DO beta and is coupled with SOCS4.

The immunomodulatory fusion protein (IFP) of the invention may further comprise a compound selected from the group consisting of CD64, CD14, CD16b, CD25, CD36, CD39, CD80, CD86, CD89, CD273, CD284, HLA-A major histocompatibility complex, class I, A, HLA-B major histocompatibility complex, class I, B, HLA-C major histocompatibility complex, class I, C, HLA-E major histocompatibility complex, class I, E, HLA-F major histocompatibility complex, class I, F, HLA-G major histocompatibility complex, class I, G, HLA-DQA1 major histocompatibility complex, class II, DQ alpha 1, HLA-DQA2 major histocompatibility complex, class II, DQ alpha 2, HLA-DQB1 major histocompatibility complex, class II, DQ beta 1, HLA-DQB2 major histocompatibility complex, class II, DQ beta 2, HLA-DRA major histocompatibility complex, class II, DR alpha HLA-DRB1 major histocompatibility complex, class II, DR beta 1, HLA-DRB3 major histocompatibility complex, class II, DR beta 3, HLA-DRB4 major histocompatibility complex, class II, DR beta 4, HLA-DRB5 major histocompatibility complex, class II, DR beta 5, CD74 molecule, major histocompatibility complex, class II invariant chain, HLA-DPA1 major histocompatibility complex, class II, DP alpha 1, HLA-DPB1 major histocompatibility complex, class II, DP beta 1, HLA-DMA major histocompatibility complex, class II, DM alpha, HLA-DMB major histocompatibility complex, class II, DM beta, HLA-DOA major histocompatibility complex, class II, DO alpha, HLA-DOB major histocompatibility complex, class II, and DO beta and is coupled with SOCS5.

The immunomodulatory fusion protein (IFP) of the invention may further comprise a compound selected from the group consisting of CD64, CD14, CD16b, CD25, CD36, CD39, CD80, CD86, CD89, CD273, CD284, HLA-A major histocompatibility complex, class I, A, HLA-B major histocompatibility complex, class I, B, HLA-C major histocompatibility complex, class I, C, HLA-E major histocompatibility complex, class I, E, HLA-F major histocompatibility complex, class I, F, HLA-G major histocompatibility complex, class I, G, HLA-DQA1 major histocompatibility complex, class II, DQ alpha 1, HLA-DQA2 major histocompatibility complex, class II, DQ alpha 2, HLA-DQB1 major histocompatibility complex, class II, DQ beta 1, HLA-DQB2 major histocompatibility complex, class II, DQ beta 2, HLA-DRA major histocompatibility complex, class II, DR alpha HLA-DRB1 major histocompatibility complex, class II, DR beta 1, HLA-DRB3 major histocompatibility complex, class II, DR beta 3, HLA-DRB4 major histocompatibility complex, class II, DR beta 4, HLA-DRB5 major histocompatibility complex, class II, DR beta 5, CD74 molecule, major histocompatibility complex, class II invariant chain, HLA-DPA1 major histocompatibility complex, class II, DP alpha 1, HLA-DPB1 major histocompatibility complex, class II, DP beta 1, HLA-DMA major histocompatibility complex, class II, DM alpha, HLA-DMB major histocompatibility complex, class II, DM beta, HLA-DOA major histocompatibility complex, class II, DO alpha, HLA-DOB major histocompatibility complex, class II, and DO beta and is coupled with SOCS6.

The immunomodulatory fusion protein (IFP) of the invention may further comprise a compound selected from the group consisting of CD64, CD14, CD16b, CD25, CD36, CD39, CD80, CD86, CD89, CD273, CD284, HLA-A major histocompatibility complex, class I, A, HLA-B major histocompatibility complex, class I, B, HLA-C major histocompatibility complex, class I, C, HLA-E major histocompatibility complex, class I, E, HLA-F major histocompatibility complex, class I, F, HLA-G major histocompatibility complex, class I, G, HLA-DQA1 major histocompatibility complex, class II, DQ alpha 1, HLA-DQA2 major histocompatibility complex, class II, DQ alpha 2, HLA-DQB1 major histocompatibility complex, class II, DQ beta 1, HLA-DQB2 major histocompatibility complex, class II, DQ beta 2, HLA-DRA major histocompatibility complex, class II, DR alpha HLA-DRB1 major histocompatibility complex, class II, DR beta 1, HLA-DRB3 major histocompatibility complex, class II, DR beta 3, HLA-DRB4 major histocompatibility complex, class II, DR beta 4, HLA-DRB5 major histocompatibility complex, class II, DR beta 5, CD74 molecule, major histocompatibility complex, class II invariant chain, HLA-DPA1 major histocompatibility complex, class II, DP alpha 1, HLA-DPB1 major histocompatibility complex, class II, DP beta 1, HLA-DMA major histocompatibility complex, class II, DM alpha, HLA-DMB major histocompatibility complex, class II, DM beta, HLA-DOA major histocompatibility complex, class II, DO alpha, HLA-DOB major histocompatibility complex, class II, and DO beta and is coupled with SOCS7.

The immunomodulatory fusion protein (IFP) of the invention may further comprise a compound selected from the group consisting of CD64, CD14, CD16b, CD25, CD36, CD39, CD80, CD86, CD89, CD273, CD284, HLA-A major histocompatibility complex, class I, A, HLA-B major histocompatibility complex, class I, B, HLA-C major histocompatibility complex, class I, C, HLA-E major histocompatibility complex, class I, E, HLA-F major histocompatibility complex, class I, F, HLA-G major histocompatibility complex, class I, G, HLA-DQA1 major histocompatibility complex, class II, DQ alpha 1, HLA-DQA2 major histocompatibility complex, class II, DQ alpha 2, HLA-DQB1 major histocompatibility complex, class II, DQ beta 1, HLA-DQB2 major histocompatibility complex, class II, DQ beta 2, HLA-DRA major histocompatibility complex, class II, DR alpha HLA-DRB1 major histocompatibility complex, class II, DR beta 1, HLA-DRB3 major histocompatibility complex, class II, DR beta 3, HLA-DRB4 major histocompatibility complex, class II, DR beta 4, HLA-DRB5 major histocompatibility complex, class II, DR beta 5, CD74 molecule, major histocompatibility complex, class II invariant chain, HLA-DPA1 major histocompatibility complex, class II, DP alpha 1, HLA-DPB1 major histocompatibility complex, class II, DP beta 1, HLA-DMA major histocompatibility complex, class II, DM alpha, HLA-DMB major histocompatibility complex, class II, DM beta, HLA-DOA major histocompatibility complex, class II, DO alpha, HLA-DOB major histocompatibility complex, class II, and DO beta and is coupled with KLF4 Kruppel-like factor 4.

Particular examples are shown in Fig. 15, Seq ID Nos. 1-33.

In another embodiment of the invention, the component A is a chemokine or a specifically binding fragment thereof. According to the invention the chemokine is in particular selected from table 1. The chemokines introduced there are the CXC chemokine/receptor family such as CXCL1, CXCL2, CXCL3, CXCL4, CXCL5, CXCL6, CXCL7, CXCL8, CXCL9, CXCL10, CXCL11, CXCL12, CXCL13, CXCL14, KXCL15, CXCL16; or the C chemokine /receptor family, such as XCL1 and XCL2; or the CX₃C chemokine/receptor family, such as CX3CL1, or the CC chemokine/receptor family, such as CCL1, CCL2, CCL3, CCL3L1, CCL4, CCLS, (CCL6), CCL7, CCL8, (CCL9/10), CCL11, (CCL12)CCL13, CCL14, CCL15, CCL16, CCL17, CCL18, CCL19, CCL20, CCL21, CCL22, CCL23, CCL24, CCL25, CCL26, CCL27, CCL28.

In another embodiment of the invention, the immunomodulatory fusion protein (IFP) of the invention is a chemokine or a specifically binding fragment thereof, in particular selected from table 1, i. e. the CXC chemokine/receptor family such as CXCL1, CXCL2, CXCL3, CXCL4, CXCL5, CXCL6, CXCL7, CXCL8, CXCL9, CXCL10, CXCL11, CXCL12, CXCL13, CXCL14, KXCL15, CXCL16; or the C chemokine /receptor family, such as XCL1 and XCL2; or the CX₃C chemokine/receptor family, such as CX3CL1, or the CC chemokine/receptor family, such as CCL1, CCL2, CCL3, CCL3L1, CCL4, CCLS, (CCL6), CCL7, CCL8, (CCL9/10), CCL11, (CCL12)CCL13, CCL14, CCL15, CCL16, CCL17, CCL18, CCL19, CCL20, CCL21, CCL22, CCL23, CCL24, CCL25, CCL26, CCL27, CCL28 coupled with a macrophage modulatory protein.

In another embodiment of the invention, the immunomodulatory fusion protein (IFP) of the invention is a chemokine or a specifically binding fragment thereof, in particular selected from table 1, i. e. the CXC chemokine/receptor family such as CXCL1, CXCL2, CXCL3, CXCL4, CXCL5, CXCL6, CXCL7, CXCL8, CXCL9, CXCL10, CXCL11, CXCL12, CXCL13, CXCL14, KXCL15, CXCL16; or the C chemokine /receptor family, such as XCL1 and XCL2; or the CX₃C chemokine/receptor family, such as CX3CL1, or the CC chemokine/receptor family, such as CCL1, CCL2, CCL3, CCL3L1, CCL4, CCLS, (CCL6), CCL7, CCL8, (CCL9/10), CCL11, (CCL12)CCL13, CCL14, CCL15, CCL16, CCL17, CCL18, CCL19, CCL20, CCL21, CCL22, CCL23, CCL24, CCL25, CCL26, CCL27, CCL28 coupled with murine C/EBPβ. In another embodiment of the invention, the immunomodulatory fusion protein (IFP) of the invention is a chemokine or a specifically binding fragment thereof, in particular selected from table 1, i. e. the CXC chemokine/receptor family such as CXCL1, CXCL2, CXCL3, CXCL4, CXCL5, CXCL6, CXCL7, CXCL8, CXCL9, CXCL10, CXCL11, CXCL12, CXCL13, CXCL14, KXCL15, CXCL16; or the C chemokine /receptor family, such as XCL1 and XCL2; or the CX₃C chemokine/receptor family, such as CX3CL1, or the CC chemokine/receptor family, such as CCL1, CCL2, CCL3, CCL3L1, CCL4, CCLS, (CCL6), CCL7, CCL8, (CCL9/10), CCL11, (CCL12)CCL13, CCL14, CCL15, CCL16, CCL17, CCL18, CCL19, CCL20, CCL21, CCL22, CCL23, CCL24, CCL25, CCL26, CCL27, CCL28 coupled with human C/EBPβ.

In another embodiment of the invention, the immunomodulatory fusion protein (IFP) of the invention is a chemokine or a specifically binding fragment thereof, in particular selected from table 1, i. e. the CXC chemokine/receptor family such as CXCL1, CXCL2, CXCL3, CXCL4, CXCL5, CXCL6, CXCL7, CXCL8, CXCL9, CXCL10, CXCL11, CXCL12, CXCL13, CXCL14, KXCL15, CXCL16; or the C chemokine /receptor family, such as XCL1 and XCL2; or the CX₃C chemokine/receptor family, such as CX3CL1, or the CC chemokine/receptor family, such as CCL1, CCL2, CCL3, CCL3L1, CCL4, CCLS, (CCL6), CCL7, CCL8, (CCL9/10), CCL11, (CCL12)CCL13, CCL14, CCL15, CCL16, CCL17, CCL18, CCL19, CCL20, CCL21, CCL22, CCL23, CCL24, CCL25, CCL26, CCL27, CCL28 coupled with Thioredoxin.

In another embodiment of the invention, the immunomodulatory fusion protein (IFP) of the invention is a chemokine or a specifically binding fragment thereof, in particular selected from table 1, i. e. the CXC chemokine/receptor family such as CXCL1, CXCL2, CXCL3, CXCL4, CXCL5, CXCL6, CXCL7, CXCL8, CXCL9, CXCL10, CXCL11, CXCL12, CXCL13, CXCL14, KXCL15, CXCL16; or the C chemokine /receptor family, such as XCL1 and XCL2; or the CX₃C chemokine/receptor family, such as CX3CL1, or the CC chemokine/receptor family, such as CCL1, CCL2, CCL3, CCL3L1, CCL4, CCLS, (CCL6), CCL7, CCL8, (CCL9/10), CCL11, (CCL12)CCL13, CCL14, CCL15, CCL16, CCL17, CCL18, CCL19, CCL20, CCL21, CCL22, CCL23, CCL24, CCL25, CCL26, CCL27, CCL28 coupled with SOCS1.

In another embodiment of the invention, the immunomodulatory fusion protein (IFP) of the invention is a chemokine or a specifically binding fragment thereof, in particular selected from table 1, i. e. the CXC chemokine/receptor family such as CXCL1, CXCL2, CXCL3, CXCL4, CXCL5, CXCL6, CXCL7, CXCL8, CXCL9, CXCL10, CXCL11, CXCL12, CXCL13, CXCL14, KXCL15, CXCL16; or the C chemokine /receptor family, such as XCL1 and XCL2; or the CX₃C chemokine/receptor family, such as CX3CL1, or the CC chemokine/receptor family, such as CCL1, CCL2, CCL3, CCL3L1, CCL4, CCLS, (CCL6), CCL7, CCL8, (CCL9/10), CCL11, (CCL12)CCL13, CCL14, CCL15, CCL16, CCL17, CCL18, CCL19, CCL20, CCL21, CCL22, CCL23, CCL24, CCL25, CCL26, CCL27, CCL28 coupled with SOCS2.

In another embodiment of the invention, the immunomodulatory fusion protein (IFP) of the invention is a chemokine or a specifically binding fragment thereof, in particular selected from table 1, i. e. the CXC chemokine/receptor family such as CXCL1, CXCL2, CXCL3, CXCL4, CXCL5, CXCL6, CXCL7, CXCL8, CXCL9, CXCL10, CXCL11, CXCL12, CXCL13, CXCL14, KXCL15, CXCL16; or the C chemokine /receptor family, such as XCL1 and XCL2; or the CX₃C chemokine/receptor family, such as CX3CL1, or the CC chemokine/receptor family, such as CCL1, CCL2, CCL3, CCL3L1, CCL4, CCLS, (CCL6), CCL7, CCL8, (CCL9/10), CCL11, (CCL12)CCL13, CCL14, CCL15, CCL16, CCL17, CCL18, CCL19, CCL20, CCL21, CCL22, CCL23, CCL24, CCL25, CCL26, CCL27, CCL28 coupled with SOCS3.

In another embodiment of the invention, the immunomodulatory fusion protein (IFP) of the invention is a chemokine or a specifically binding fragment thereof, in particular selected from table 1, i. e. the CXC chemokine/receptor family such as CXCL1, CXCL2, CXCL3, CXCL4, CXCL5, CXCL6, CXCL7, CXCL8, CXCL9, CXCL10, CXCL11, CXCL12, CXCL13, CXCL14, KXCL15, CXCL16; or the C chemokine /receptor family, such as XCL1 and XCL2; or the CX₃C chemokine/receptor family, such as CX3CL1, or the CC chemokine/receptor family, such as CCL1, CCL2, CCL3, CCL3L1, CCL4, CCLS, (CCL6), CCL7, CCL8, (CCL9/10), CCL11, (CCL12)CCL13, CCL14, CCL15, CCL16, CCL17, CCL18, CCL19, CCL20, CCL21, CCL22, CCL23, CCL24, CCL25, CCL26, CCL27, CCL28 coupled with SOCS4.

In another embodiment of the invention, the immunomodulatory fusion protein (IFP) of the invention is a chemokine or a specifically binding fragment thereof, in particular selected from table 1, i. e. the CXC chemokine/receptor family such as CXCL1, CXCL2, CXCL3, CXCL4, CXCL5, CXCL6, CXCL7, CXCL8, CXCL9, CXCL10, CXCL11, CXCL12, CXCL13, CXCL14, KXCL15, CXCL16; or the C chemokine /receptor family, such as XCL1 and XCL2; or the CX₃C chemokine/receptor family, such as CX3CL1, or the CC chemokine/receptor family, such as CCL1, CCL2, CCL3, CCL3L1, CCL4, CCLS, (CCL6), CCL7, CCL8, (CCL9/10), CCL11, (CCL12)CCL13, CCL14, CCL15, CCL16, CCL17, CCL18, CCL19, CCL20, CCL21, CCL22, CCL23, CCL24, CCL25, CCL26, CCL27, CCL28 coupled with SOCS5.

In another embodiment of the invention, the immunomodulatory fusion protein (IFP) of the invention is a chemokine or a specifically binding fragment thereof, in particular selected from table 1, i. e. the CXC chemokine/receptor family such as CXCL1, CXCL2, CXCL3, CXCL4, CXCL5, CXCL6, CXCL7, CXCL8, CXCL9, CXCL10, CXCL11, CXCL12, CXCL13, CXCL14, KXCL15, CXCL16; or the C chemokine /receptor family, such as XCL1 and XCL2; or the CX₃C chemokine/receptor family, such as CX3CL1, or the CC chemokine/receptor family, such as CCL1, CCL2, CCL3, CCL3L1, CCL4, CCLS, (CCL6), CCL7, CCL8, (CCL9/10), CCL11, (CCL12)CCL13, CCL14, CCL15, CCL16, CCL17, CCL18, CCL19, CCL20, CCL21, CCL22, CCL23, CCL24, CCL25, CCL26, CCL27, CCL28 coupled with SOCS6.

In another embodiment of the invention, the immunomodulatory fusion protein (IFP) of the invention is a chemokine or a specifically binding fragment thereof, in particular selected from table 1, i. e. the CXC chemokine/receptor family such as CXCL1, CXCL2, CXCL3, CXCL4, CXCL5, CXCL6, CXCL7, CXCL8, CXCL9, CXCL10, CXCL11, CXCL12, CXCL13, CXCL14, KXCL15, CXCL16; or the C chemokine /receptor family, such as XCL1 and XCL2; or the CX₃C chemokine/receptor family, such as CX3CL1, or the CC chemokine/receptor family, such as CCL1, CCL2, CCL3, CCL3L1, CCL4, CCLS, (CCL6), CCL7, CCL8, (CCL9/10), CCL11, (CCL12)CCL13, CCL14, CCL15, CCL16, CCL17, CCL18, CCL19, CCL20, CCL21, CCL22, CCL23, CCL24, CCL25, CCL26, CCL27, CCL28 coupled with SOCS7.

In another embodiment of the invention, the immunomodulatory fusion protein (IFP) of the invention is a chemokine or a specifically binding fragment thereof, in particular selected from table 1, i. e. the CXC chemokine/receptor family such as CXCL1, CXCL2, CXCL3, CXCL4, CXCL5, CXCL6, CXCL7, CXCL8, CXCL9, CXCL10, CXCL11, CXCL12, CXCL13, CXCL14, KXCL15, CXCL16; or the C chemokine /receptor family, such as XCL1 and XCL2; or the CX₃C chemokine/receptor family, such as CX3CL1, or the CC chemokine/receptor family, such as CCL1, CCL2, CCL3, CCL3L1, CCL4, CCLS, (CCL6), CCL7, CCL8, (CCL9/10), CCL11, (CCL12)CCL13, CCL14, CCL15, CCL16, CCL17, CCL18, CCL19, CCL20, CCL21, CCL22, CCL23, CCL24, CCL25, CCL26, CCL27, CCL28 coupled with KLF4 Kruppel-like factor 4.

In another embodiment of the invention, component A is an interleukin or a specifically binding fragment thereof like those provided in table 2 binding to its specific cellular receptor, i. e the immunomodulatory fusion protein (IFP) of the invention is formed by the combination of the following interleukins: IL-1; IL-2; IL-3; IL-4; IL-5; IL-6; IL-7; IL-8; IL-9; IL-10; IL-11; IL-12; IL-13; IL-14; IL-15; IL-16; IL-17; IL-18; IL-19; IL-20; IL-21; IL-22; IL-23; IL-24; IL-25; IL-26; IL-27; IL-28; IL-29; IL-30; IL-31; IL-32; IL-33; IL-35; and a macrophage modulatory protein.

In another embodiment of the invention, component A is an interleukin or a specifically binding fragment thereof like those provided in table 2 binding to its specific cellular receptor, i. e the immunomodulatory fusion protein (IFP) of the invention is formed by the combination of the following interleukins: IL-1; IL-2; IL-3; IL-4; IL-5; IL-6; IL-7; IL-8; IL-9; IL-10; IL-11; IL-12; IL-13; IL-14; IL-15; IL-16; IL-17; IL-18; IL-19; IL-20; IL-21; IL-22; IL-23; IL-24; IL-25; IL-26; IL-27; IL-28; IL-29; IL-30; IL-31; IL-32; IL-33; IL-35; and murine C/EBPβ.

In another embodiment of the invention, component A is an interleukin or a specifically binding fragment thereof like those provided in table 2 binding to its specific cellular receptor, i. e the immunomodulatory fusion protein (IFP) of the invention is formed by the combination of the following interleukins: IL-1; IL-2; IL-3; IL-4; IL-5; IL-6; IL-7; IL-8; IL-9; IL-10; IL-11; IL-12; IL-13; IL-14; IL-15; IL-16; IL-17; IL-18; IL-19; IL-20; IL-21; IL-22; IL-23; IL-24; IL-25; IL-26; IL-27; IL-28; IL-29; IL-30; IL-31; IL-32; IL-33; IL-35; and human C/EBPβ.

In another embodiment of the invention, component A is an interleukin or a specifically binding fragment thereof like those provided in table 2 binding to its specific cellular receptor, i. e the immunomodulatory fusion protein (IFP) of the invention is formed by the combination of the following interleukins: IL-1; IL-2; IL-3; IL-4; IL-5; IL-6; IL-7; IL-8; IL-9; IL-10; IL-11; IL-12; IL-13; IL-14; IL-15; IL-16; IL-17; IL-18; IL-19; IL-20; IL-21; IL-22; IL-23; IL-24; IL-25; IL-26; IL-27; IL-28; IL-29; IL-30; IL-31; IL-32; IL-33; IL-35; and Thioredoxin.

In another embodiment of the invention, component A is an interleukin or a specifically binding fragment thereof like those provided in table 2 binding to its specific cellular receptor, i. e the immunomodulatory fusion protein (IFP) of the invention is formed by the combination of the following interleukins: IL-1; IL-2; IL-3; IL-4; IL-5; IL-6; IL-7; IL-8; IL-9; IL-10; IL-11; IL-12; IL-13; IL-14; IL-15; IL-16; IL-17; IL-18; IL-19; IL-20; IL-21; IL-22; IL-23; IL-24; IL-25; IL-26; IL-27; IL-28; IL-29; IL-30; IL-31; IL-32; IL-33; IL-35; and SOCS1.

In another embodiment of the invention, component A is an interleukin or a specifically binding fragment thereof like those provided in table 2 binding to its specific cellular receptor, i. e the immunomodulatory fusion protein (IFP) of the invention is formed by the combination of the following interleukins: IL-1; IL-2; IL-3; IL-4; IL-5; IL-6; IL-7; IL-8; IL-9; IL-10; IL-11; IL-12; IL-13; IL-14; IL-15; IL-16; IL-17; IL-18; IL-19; IL-20; IL-21; IL-22; IL-23; IL-24; IL-25; IL-26; IL-27; IL-28; IL-29; IL-30; IL-31; IL-32; IL-33; IL-35; and SOCS2.

In another embodiment of the invention, component A is an interleukin or a specifically binding fragment thereof like those provided in table 2 binding to its specific cellular receptor, i. e the immunomodulatory fusion protein (IFP) of the invention is formed by the combination of the following interleukins: IL-1; IL-2; IL-3; IL-4; IL-5; IL-6; IL-7; IL-8; IL-9; IL-10; IL-11; IL-12; IL-13; IL-14; IL-15; IL-16; IL-17; IL-18; IL-19; IL-20; IL-21; IL-22; IL-23; IL-24; IL-25; IL-26; IL-27; IL-28; IL-29; IL-30; IL-31; IL-32; IL-33; IL-35; and SOCS3.

In another embodiment of the invention, component A is an interleukin or a specifically binding fragment thereof like those provided in table 2 binding to its specific cellular receptor, i. e the immunomodulatory fusion protein (IFP) of the invention is formed by the combination of the following interleukins: IL-1; IL-2; IL-3; IL-4; IL-5; IL-6; IL-7; IL-8; IL-9; IL-10; IL-11; IL-12; IL-13; IL-14; IL-15; IL-16; IL-17; IL-18; IL-19; IL-20; IL-21; IL-22; IL-23; IL-24; IL-25; IL-26; IL-27; IL-28; IL-29; IL-30; IL-31; IL-32; IL-33; IL-35; and SOCS4.

In another embodiment of the invention, component A is an interleukin or a specifically binding fragment thereof like those provided in table 2 binding to its specific cellular receptor, i. e the immunomodulatory fusion protein (IFP) of the invention is formed by the combination of the following interleukins: IL-1; IL-2; IL-3; IL-4; IL-5; IL-6; IL-7; IL-8; IL-9; IL-10; IL-11; IL-12; IL-13; IL-14; IL-15; IL-16; IL-17; IL-18; IL-19; IL-20; IL-21; IL-22; IL-23; IL-24; IL-25; IL-26; IL-27; IL-28; IL-29; IL-30; IL-31; IL-32; IL-33; IL-35; and SOCS5.

In another embodiment of the invention, component A is an interleukin or a specifically binding fragment thereof like those provided in table 2 binding to its specific cellular receptor, i. e the immunomodulatory fusion protein (IFP) of the invention is formed by the combination of the following interleukins: IL-1; IL-2; IL-3; IL-4; IL-5; IL-6; IL-7; IL-8; IL-9; IL-10; IL-11; IL-12; IL-13; IL-14; IL-15; IL-16; IL-17; IL-18; IL-19; IL-20; IL-21; IL-22; IL-23; IL-24; IL-25; IL-26; IL-27; IL-28; IL-29; IL-30; IL-31; IL-32; IL-33; IL-35; and SOCS6.

In another embodiment of the invention, component A is an interleukin or a specifically binding fragment thereof like those provided in table 2 binding to its specific cellular receptor, i. e the immunomodulatory fusion protein (IFP) of the invention is formed by the combination of the following interleukins: IL-1; IL-2; IL-3; IL-4; IL-5; IL-6; IL-7; IL-8; IL-9; IL-10; IL-11; IL-12; IL-13; IL-14; IL-15; IL-16; IL-17; IL-18; IL-19; IL-20; IL-21; IL-22; IL-23; IL-24; IL-25; IL-26; IL-27; IL-28; IL-29; IL-30; IL-31; IL-32; IL-33; IL-35; and SOCS7.

In another embodiment of the invention, component A is an interleukin or a specifically binding fragment thereof like those provided in table 2 binding to its specific cellular receptor, i. e the immunomodulatory fusion protein (IFP) of the invention is formed by the combination of the following interleukins: IL-1; IL-2; IL-3; IL-4; IL-5; IL-6; IL-7; IL-8; IL-9; IL-10; IL-11; IL-12; IL-13; IL-14; IL-15; IL-16; IL-17; IL-18; IL-19; IL-20; IL-21; IL-22; IL-23; IL-24; IL-25; IL-26; IL-27; IL-28; IL-29; IL-30; IL-31; IL-32; IL-33; IL-35; and KLF4 Kruppel-like factor 4.

The component B of the immunomodulatory fusion protein (IFP) of the invention is murine C/EBPβ, or human C/EBPβ. Representative examples of nucleotides coding for amino acid sequences of component B are listed as Seq ID Nos. 34 - 44 in Fig. 16.

In yet a further embodiment the immunomodulatory fusion protein (IFP) of the invention comprises or consists of the components A, and/or B are chemically coupled or fused to each other by genetic engineering, such as exemplified by the proteins having one of the amino acid sequences encoded by the polynucleotide of one of the Seq ID Nos. 45 - 49 (Fig. 17).

Another subject matter of the invention is a process for manufacturing the immunomodulatory fusion protein (IFP) of the invention by
- cloning a component B to yield a polynucleotide; and
- fuse said polynucleotide coding for component B with a polynucleotide coding for a protein of component A to yield a polynucleotide coding for the immunomodulatory fusion protein (IFP) of the invention;
- expressing said polynucleotide coding for the immunomodulatory fusion protein (IFP) of the invention in suitable host, such as *E. coli;*
- isolation and purification of the immunomodulatory fusion protein (IFP) of the invention.

Subject matter of the invention is further a vector comprising or consisting of at least one polynucleotide of the invention and a cell having the vector of the invention.

Further a medicament comprising or consisting of the immunomodulatory fusion protein (IFP) of the invention is also subject matter of the invention as well as a composition of matter comprising or consisting of the immunomodulatory fusion protein (IFP) of the invention for use in the treatment of chronic inflammatory diseases.

The immunomodulatory fusion protein (IFP) according to the invention is a compound comprising or consisting of at least two domains, i.e. one domain having a macrophage modulatory protein and at least one cell-specific binding domain. The immunomodulatory fusion protein (IFP) according to the invention is usable for therapy of chronic inflammatory diseases.

As used herein, the term "immunotoxin" refers to chimeric molecules in which a cell-binding monoclonal antibody or fragments thereof are chemically coupled or genetically fused to a bacterial or plant protein-based toxin. Immunotoxins *per* se as well as their constructions are well known to the person skilled in the art.

As used herein, the term "immunomodulatory fusion protein" refers to a chimeric or completely human bi-functional fusion protein in which a cell-binding human ligand or fragments thereof are chemically coupled or genetically fused to a macrophage modulatory protein or at least a partial sequence of the macrophage modulatory protein, the partial sequence having maintained the modulatory function.

As used herein, the term "component A" of the immunomodulatory fusion protein (IFP) represents the macrophage cell-specific binding structure of the immunomodulatory fusion protein (IFP) of present invention. The component A is selected from the group of internalizing cell surface receptor binding structures consisting of antibodies or their derivatives or fragments thereof, synthetic peptides such as scFv, mimotopes, etc. or chemical molecules such as carbohydrates, lipids, nucleic acids, peptides, vitamins, etc., and/or small molecules with up to 100 atoms with receptor-binding activity like ligands, in particular single atoms, peptidic molecules, non-peptidic molecules, etc., and/or carbohydrate binding proteins and their ligands such as lectins, in particular calnexins, c-type lectins, I-type lectins, m-type lectins, p-type lectins, r-type lectins, galectins and their derivatives, and/or receptor binding molecules such as natural ligands to the cluster of differentiation (CD) antigens, like CD30, CD40, etc., cytokines such as chemokines, colony stimulating factors, type-1 cytokines, type-2 cytokines, interferons, interleukins, lymphokines, monokines, etc., and/or adhesion molecules including their derivatives and mutants, and/or derivatives or combinations of any of the above listed binding structures.

In an additional object of the present invention, component A is binding to a cell surface marker of a healthy or diseased cell belonging to the cluster of differentiation antigens and major histocompatibility complex antigens (Fig. 15, Seq ID Nos. 1-33).

In another specific embodiment, the component A is a chemokine or a specifically binding fragment thereof like those provided in table 1 binding to its specific cellular receptors.

In another embodiment, component A is an interleukin or a specifically binding fragment thereof like those provided in table 2 binding to its specific cellular receptor.

As used herein, the term "antibody" refers to polyclonal antibodies, monoclonal antibodies, humanized antibodies, single-chain antibodies, and fragments thereof such as Fab, F(ab')2, Fv, and other fragments which retain the antigen binding function and specificity of the parent antibody.

As used herein, the term "monoclonal antibody" refers to an antibody composition having a homogeneous antibody population. The term is not limited regarding the species or source of the antibody, nor is it intended to be limited by the manner in which it is made. The term encompasses whole immunoglobulins as well as fragments such as Fab, F(ab')2, Fv, and others which retain the antigen binding function and specificity of the antibody. Monoclonal antibodies of any mammalian species can be used in this invention. In practice, however, the antibodies will typically be of rat or murine origin because of the availability of rat or murine cell lines for use in making the required hybrid cell lines or hybridomas to produce monoclonal antibodies.

As used herein, the term "human antibodies" means that the framework regions of an immunoglobulin are derived from human immunoglobulin sequences.

As used herein, the term "single chain antibody fragments" (scFv) refers to antibodies prepared by determining the binding domains (both heavy and light chains) of a binding antibody, and supplying a linking moiety, which permits preservation of the binding function. This forms, in essence, a radically abbreviated antibody, having only that part of the variable domain necessary for binding to the antigen. Determination and construction of single chain antibodies are described in U.S. Pat. No. 4,946,778 to Ladner et al.

The term "target cell" refers to cells carrying an extracellular surface structure to which the component A of the immunomodulatory fusion protein (IFP) actively or passively binds. Target cells are thus cells to which the component A of the immunomodulatory fusion protein (IFP) can bind. The target cells are further characterized by their ability to internalize the immunomodulatory fusion protein (IFP) according to the present invention upon binding of component A. The target cells are further characterized by their pro-inflammatory (M1 or M1-like) polarization status.

The term "soluble" refers to the ability of the immunomodulatory fusion protein (IFP) to stay in solution when recombinantly expressed, in particular during protein purification, enabling high yields. The term "soluble" also refers to the state of the immunomodulatory fusion protein (IFP) in fluidic systems inside an organism, until specifically attached to the target cell/tissue. The term also refers to the state of the immunomodulatory fusion protein (IFP) inside a cell upon release from any kind of incorporation vesicles.

The term "endogenous" refers to the localization of the immunomodulatory fusion protein (IFP) in the surrounding/environment of a given target cell.

The term "synthetic" refers to a man-made immunomodulatory fusion protein (IFP), not found in nature. The term also comprises or consists of the meaning of "recombinant".

The term "recombinant" refers to the preparation of molecules, in particular the covalent joining of molecules from different sources, by any one of the known methods of molecular biology. As used in the present invention, the term "recombinant" refers in particular to the fusion of the antibody part to the protein, which has modulatory activity, by any one of the known methods of molecular biology, such as through production of single chain antibodies.

The recombinant DNA molecule encoding the recombinant immunomodulatory fusion protein (IFP) - in this case a fusion protein - comprising or consisting of the antibody part and the protein, which has modulatory activity, are recombinantly expressed. Recombinant immunomodulatory fusion proteins produced in this way may be isolated by any technique known in the field of recombinant DNA expression technology suitable for this purpose.

The immunomodulatory fusion protein (IFP) of the invention can be a soluble, endogenous, synthetic and/or recombinantly manufactured immunomodulatory fusion protein (IFP).

The term "derivative" refers to a mutated or modified protein, which has retained its characterizing activity, i.e. binding activity or microtubules stabilizing activity. Particular preferred are constitutively active derivatives. The term derivative comprises or consists of proteins, which carry at least one amino acid substitution, deletion, addition, a swapping of a single domain or at least one modification of at least one amino acid. Preferred are derivatives, which carry 20 such changes, more preferred are those with 10 such changes and most preferred are those with 1 to 5 such changes. Modifications, which can occur, are phosphorylation, acetylation, methylation, prenylation and sulfation.

As used herein, the term "vector" comprises or consists of DNA and RNA forms of a plasmid, a cosmid, a phage, phagemid, derivatives of them, or a virus. A vector comprises or consists of control sequences and coding sequences.

The term "expression of the recombinant genes encoding the recombinant immunomodulatory fusion protein (IFP)", wherein the recombinant immunomodulatory fusion protein (IFP) is a single chain antibody-toxin moiety fusion polypeptide, refers to the transformation and/or transfection of a host cell with a nucleic acid or vector encoding such a immunomodulatory fusion protein (IFP), and culturing said host cells selected from the group of bacteria, such as *E. coli, and*/*or* in yeast, such as in *S*. *cerevisiae,* and/or in established mammalian or insect cell lines, such as CHO, COS, BHK, 293T and MDCK cells, and/or in primary cells, such as human cells, non-human vertebrate cells, and/or in invertebrate cells such as insect cells, and the synthesis and translation of the corresponding mRNA, finally giving rise to the recombinant protein, the recombinant immunomodulatory fusion protein (IFP). In more detail, the term "expression of the recombinant genes encoding the recombinant immunomodulatory fusion protein (IFP)", comprises or consists of the following steps:
Transformation of an appropriate cellular host with a recombinant vector, in which a nucleotide sequence coding for the fusion protein had been inserted under the control of the appropriate regulatory elements, particularly a promoter recognized by the polymerases of the cellular host. In the case of a prokaryotic host, an appropriate ribosome-binding site (RBS) also precedes the nucleotide sequence coding for the fusion protein, enabling the translation in said cellular host. In the case of a eukaryotic host any artificial signal sequence or pre/pro sequence may be provided, or the natural signal sequence may be employed. The transformed cellular host is cultured under conditions enabling the expression of said insert.

The immunomodulatory fusion protein (IFP) of the invention, in particular soluble, endogenous immunomodulatory fusion protein (IFP) can also be manufactured by chemically linking a representative of component A with one of component B. The skilled person has an arsenal of methods at his or her disposal to chemically combine two proteins. It is e.g. possible to directly couple component A and B, however, for reasons known to the skilled person when considering coupling reactions and proteins to be coupled, he or she provides for a "spacer" between the two components. The spacer between the two proteins is advantageous since the two proteins are kept apart to some extent e.g. reducing possible sterical interaction between the two components.

The term "supplementary component S", refers to an additional component of the immunomodulatory fusion protein (IFP) comprising or consisting of A and B. The supplementary component S contributes features and properties to the immunomodulatory fusion protein (IFP), which allow efficient preparation and/or modify the effectiveness of the immunomodulatory fusion protein (IFP):
- translocation of the macrophage modulatory protein into the target cells (e.g., translocation domain, membrane transfer peptides, amphiphatic sequences);
- intracellular activation/separation of component B (intracellular proteases).

Thus the supplementary component S is selected from the group of translocation domain, membrane transfer peptides, amphiphatic sequences and consensus sequences for intracellular proteases, as exemplified elsewhere [17, 18].

The invention also relates to nucleic acid molecules, such as DNA and/or RNA, or vectors, which code for the immunomodulatory fusion protein (IFP) of the present invention for preparing the immunomodulatory fusion protein (IFP). The feasibility of the expression of the nucleic acids encoding a recombinant immunomodulatory fusion protein (IFP) in eukaryotic cells of human origin is successfully documented here, as well as the feasibility to use the immunomodulatory fusion protein (IFP) as a specific agent in eukaryotic cells of human origin for interaction to microtubules. This suggests the suitability of nucleic acids coding for a immunomodulatory fusion protein (IFP) according to the invention also for non germ line gene-therapeutic approaches. A person skilled in the art is capable of recognizing the various aspects and possibilities of gene-therapeutic interventions in connection with the various diseases to be treated.

Also claimed are cells or *in vitro* translation systems, which synthesize complete immunomodulatory fusion proteins (IFP) according to the invention or individual components thereof, after transformation and/or transfection with, or addition of the nucleic acid molecules or vectors according to the invention.

Cells or organisms according to the invention are either of prokaryotic origin, especially from *E. coli, B. subtilis, S. carnosus, S. coelicolor, Marinococcus sp.,* or eukaryotic origin, especially from *Saccharomyces sp., Aspergillus sp., Spodoptera sp., P. pastoris,* primary or cultivated mammalian cells, eukaryotic cell lines (e.g., CHO, Cos or 293) or plants (e.g. *N. tabacum*).

The invention also relates to medicaments comprising or consisting of the immunomodulatory fusion protein (IFP) according to the present invention and/or the nucleic acid or vectors encoding the immunomodulatory fusion protein (IFP) of present invention. Typically, the immunomodulatory fusion proteins (IFP) according to the invention are administered in physiologically acceptable dosage forms. These include, for example, Tris, NaCl, phosphate buffers and all approved buffer systems, especially including buffer systems, which are characterized by the addition of approved protein stabilizers. The administration is effected, in particular, by parenteral, intravenous, subcutaneous, intramuscular, intratumoral, transnasal administrations, and by transmucosal application.

The dosage of the immunomodulatory fusion protein (IFP) according to the invention to be administered must be established for each application in each disease to be newly treated by clinical phase I studies (dose-escalation studies).

Nucleic acids or vectors, which code for a immunomodulatory fusion protein (IFP) according to the invention, are advantageously administered in physiologically acceptable dosage forms. These include, for example, Tris, NaCl, phosphate buffers and all approved buffer systems, especially including buffer systems, which are characterized by the addition of approved stabilizers for the nucleic acids and/or vectors to be used. The administration is effected, in particular, by parenteral, intravenous, subcutaneous, intramuscular, intratumoral, transnasal administrations, and by transmucosal application.

The immunomodulatory fusion protein (IFP) according to the invention, nucleic acid molecules coding therefore and/or cells or *in vitro* translation systems can be used for the preparation of a medicament for treating chronic inflammatory diseases.

The invention is further described - by way of example- by the cloning of a selected macrophage modulatory protein candidate (C/EBPβ) as fusion to H22(scFv) specific for binding to CD64, which is up-regulated on M1-polarized macrophages, and expressed this immunomodulatory fusion protein in *E. coli.* After affinity purification, identity and purity of obtained proteins were confirmed by SDS-PAGE followed by Coomassie staining and Western Blotting. Specific binding was verified by flow cytometry. Using CD64 as a gateway example, the potential of H22(scFv)-C/EBPβ to switch pro-inflammatory M1 macrophages to an anti-inflammatory M2-like phenotype was demonstrated *in vitro.* Together, these results suggest that targeting appropriate surface receptors of M1 macrophages using immunomodulatory fusion molecules is a promising approach for the development of novel intervention strategies for most chronic inflammatory diseases.

The invention is further illustrated by the following nonlimiting examples.

### Examples

### Phenotypic characterization of polarized macrophages

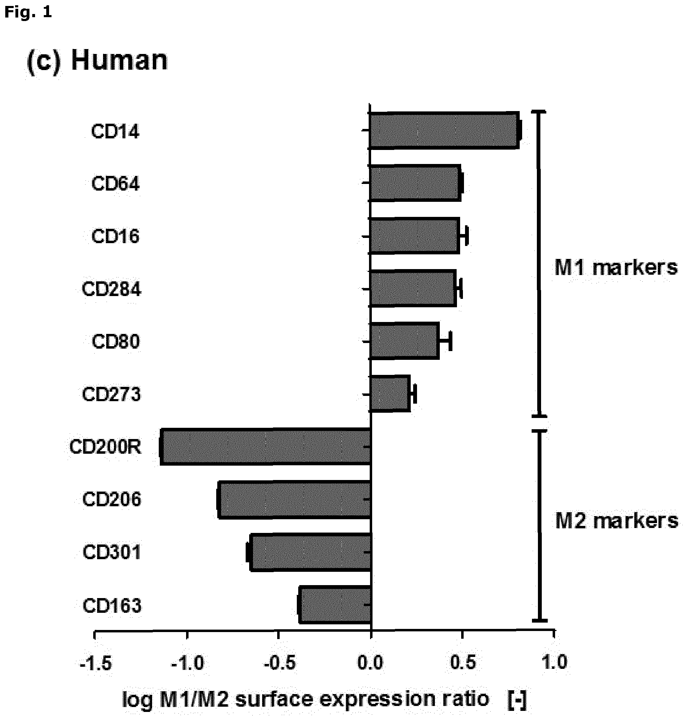

The relative expression levels of a number of surface proteins on transgenic murine macrophages were analysed expressing human CD64 (hCD64tg), polarized *in vitro* by stimulation with IFN-γ + LPS (M1) or IL-4 (M2). Eight receptors were found to be upregulated in M1 macrophages, including the human FcyRI (hCD64), the co-receptor for LPS (CD14), MHCI and MHCII (Fig. 1b). In contrast, M2 macrophages expressed lower levels of both hCD64 and CD14, but expressed nine other receptors at higher levels than M1 macrophages, including the mannose receptor (CD206) and the macrophage galactose-type C-type lectin (CD301) (Fig. 1b). The expression profiles of surface markers on human macrophages were also investigated, selecting appropriate markers from the literature. The upregulation of CD64 and CD14 in human M1 macrophages was similar to murine cells, and human M2 macrophages also showed elevated levels of CD206 and CD301 but lower levels of CD64 and CD14 (Fig. 1c). Some of these markers have already been assigned to M1 or M2 macrophages, but a set of markers were identified that allowed for discrimination between the M1 and M2 subpopulations *in vitro* and *in vivo* (Fig. 1b and c).

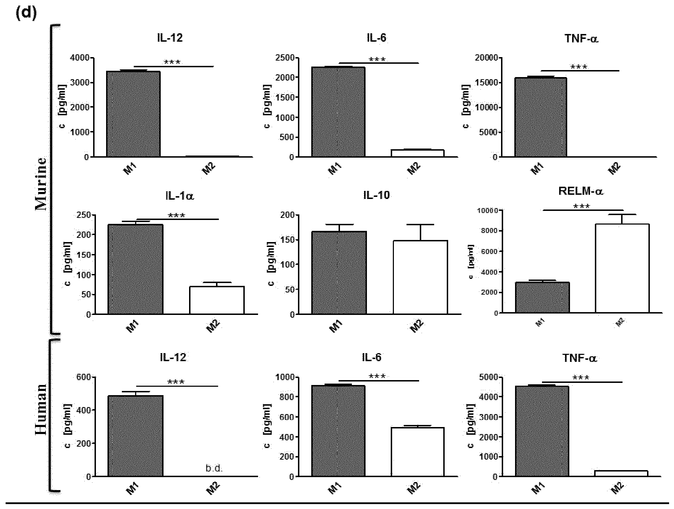

Polarization also induced changes in the expression of soluble cytokines. Whereas both murine and human M1 macrophages secreted the pro-inflammatory cytokine IL-12, this molecule was not produced by M2 macrophages. IL-6 and TNF-α were also strongly upregulated in M1 macrophages, whereas the murine M2 marker RELM-α was over 2-fold more abundant in M2 compared to M1 macrophages. The anti-inflammatory cytokine IL-10 which is known to mediate resolution [19] was detected at comparable levels in both macrophage populations (Fig. 1d).

### Elimination of M1 macrophages in vitro

After defining the M1 and M2 expression profiles and confirming the preferential expression of hCD64 by M1 macrophages, the manipulation of these cells by targeting hCD64 was investigated. It has thus far proven impossible to target M1 macrophages without adversely affecting the anti-inflammatory M2 cell population. Using the hCD64-specific single chain antibody H22(scFv) as a targeting component, the bacterial immunotoxin H22(scFv)-ETA' was shown to kill the hCD64tg M1 macrophages selectively, affecting neither the M2 nor the non-polarized populations. H22(scFv) alone without the toxin had no effect on any of the macrophage populations (Fig. 2).

Next to this, the immunotoxin on human macrophage subpopulations with identical results were also tested. Furthermore, two fully-human H22(scFv)-based cytolytic fusion proteins, H22(scFv)-Angiogenin and Granzyme B-H22(scFv), also selectively killed the human M1 macrophages showing that the cytotoxic specificity is independent of the effector protein. Staining with AnnexinV-FITC after incubation with H22(scFv)-ETA' confirmed the mode of action as the selective induction of apoptosis in M1 macrophages (Fig. 3).

### Elimination of M1 macrophages in a murine model of chronic cutaneous inflammation

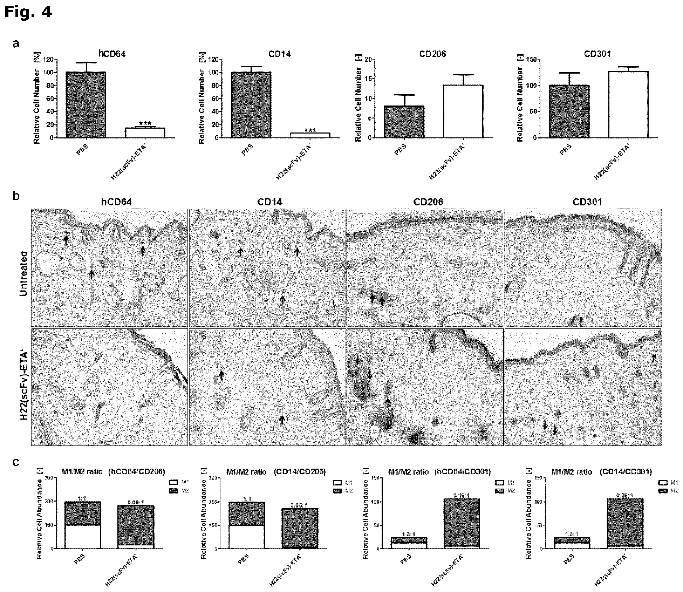

The specific elimination of M1 inflammatory macrophages was confirmed *in vivo* using a previously established sodium lauryl sulfate (SLS)-induced cutaneous inflammation model in hCD64tg mice. Topical application of SLS resulted in local skin inflammation with a predominance of M1 macrophages. Local intradermal treatment with H22(scFv)-ETA' resulted in the reduction of hCD64⁺ CD14⁺ (M1) macrophages, whereas CD206⁺ CD301⁺ (M2) macrophages remained unaffected (Fig. 4a and b). Removing M1 macrophages from the site of inflammation led to an M2-biased microenvironment, shifting the M1/M2 ratio towards an overall anti-inflammatory status (Fig. 4c). This was confirmed for all combinations (hCD64/CD206, hCD64/CD301, CD14/CD206 and CD14/CD301) of M1- and M2-specific surface markers.

### Ex vivo treatment of a chronically-inflamed skin biopsy from an atopic dermatitis patient

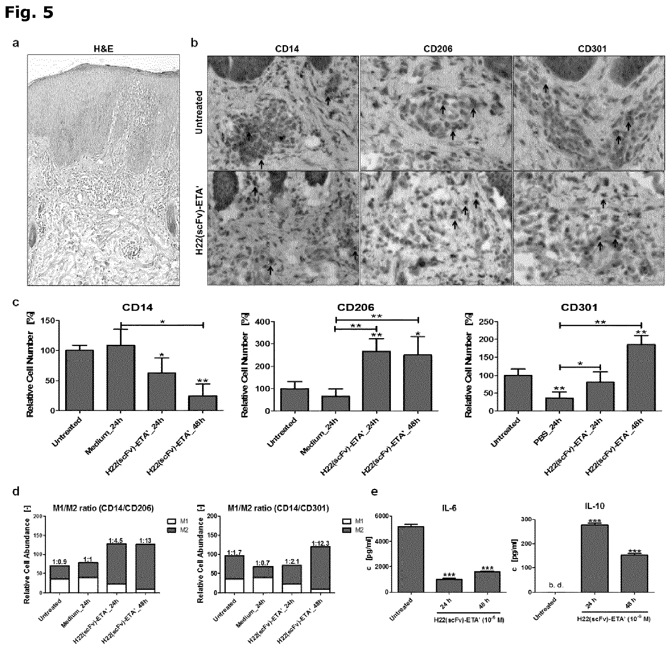

To investigate the potential clinical relevance of our findings, a skin biopsy were treated taken from lesional skin a human atopic dermatitis patient *ex vivo* using H22(scFv)-ETA'. Histological hematoxylin and eosin (H&E) staining confirmed the presence of inflammatory cells (Fig. 5a). In line with the data from the murine model, H22(scFv)-ETA' proved effective in reducing the number of M1 macrophages (CD14⁺), while significantly increasing the number of M2 macrophages (CD206⁺ and CD301⁺) (Fig. 5b and c). The M1/M2 ratio was therefore shifted towards the M2 population (Fig. 5d) as confirmed by both combinations (CD14/CD206 and CD14/CD301) of specific markers. In addition to surface receptors, the impact of H22(scFv)-ETA' on the production of soluble cytokines were also analyzed. Supernatants from the human skin biopsy were analyzed for the presence of relevant cytokines before and after treatment. Although the concentrations of IL-12, IFN-γ, TNF-α and IL-4 were below the detection limit, one was able to measure the levels of the pro-inflammatory cytokine IL-6 and the anti-inflammatory cytokine IL-10. In agreement with the distribution of surface receptors, IL-6 levels had declined sharply 24 h after treatment with H22(scFv)-ETA', whereas IL-10 levels had increased significantly. IL-10 was not detected in the untreated control (Fig. 5e).

### Phenotypic plasticity of polarized macrophages

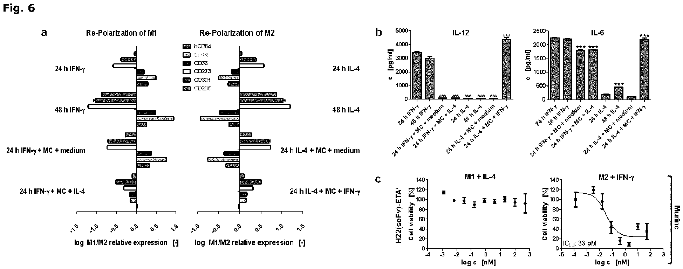

Phenotypic and functional plasticity is a key feature of polarized macrophages, and the pathological inability of macrophages to switch phenotype from M1 to M2 can lead to chronic inflammation [20]. The ability of murine peritoneal macrophages to be re-polarized by reversion of the initial stimuli (IL-4 for M1 and IFN-γ for M2) were analyzed and it was found evidence of phenotypic switching using our panel of specific surface markers. Although M1 polarized macrophages initially expressed only low levels of M2 markers (CD273, CD206 and CD301), their expression was strongly induced following the removal of the M1 stimulus and its substitution with the M2 stimulus. The reciprocal process was observed when M2 polarized macrophages were exposed to the M1 stimulus (Fig. 6a). The switch in surface receptor expression induced by IFN-γ and IL-4 respectively provided evidence that the cells respond by modulating their receptor profile. However, the critical and adverse activity of M1 macrophages during chronic inflammation is the production of pro-inflammatory cytokines, which amplify the inflammatory response, for instance by activating Th1 cells. Therefore, supernatants from the re-polarized macrophages we are re analyzed for the critical soluble cytokines IL-12 and IL-6. The production of IL-12 by M1 cells could be completely abrogated by re-polarization with IL-4. IL-6 levels were also reduced, albeit to a much lower extent. In contrast, the expression of IL-12 and IL-6 could be strongly induced in M2 cells by stimulation with IFN-γ (Fig. 6b).

### Reversible susceptibility of polarized macrophages

We were also able to demonstrate the functional plasticity of polarized macrophages by reversing their sensitivity toward immunotoxin H22(scFv)-ETA'. The stimulation of previously sensitive M1 macrophages with IL-4 resulted in the development of resistance to H22(scFv)-ETA', whereas the stimulation of previously resistant M2 macrophages with IFN-γ resulted in the development of sensitivity towards H22(scFv)-ETA' (Fig. 6c).

### Targeted modulation of M1 macrophages

The selection of protein candidates capable of switching M1 macrophages towards a non-inflammatory phenotype is based on identifying proteins critically involved in activation of gene transcription of pro-inflammatory cytokines. Two key players are responsible for the regulation of this process: NF-κB and STAT1. These proteins are part of a complex signal transduction pathway and decreased concentrations of nuclear NF-κB and phosphorylated (activated) STAT1 have been associated with reduced inflammatory potential. To intervene into the regulation of these factors, the CCAAT-enhancer binding protein β (C/EBPβ) was selected as potential immunomodulator of M1 macrophages.

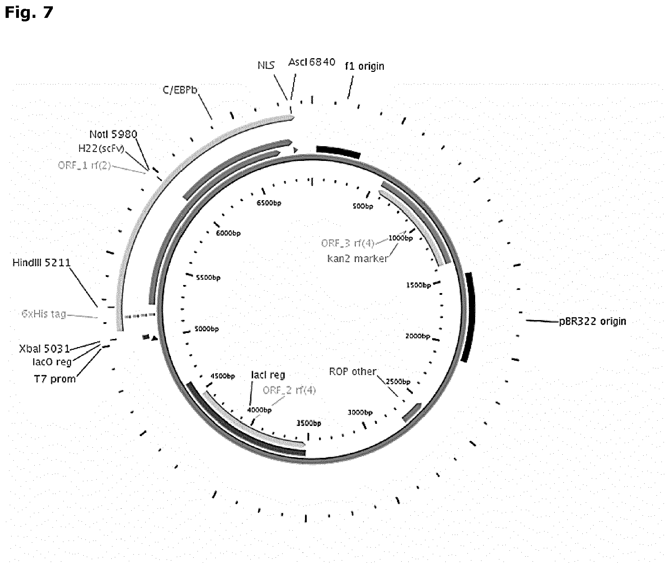

C/EBPβ is not able to enter the cell without receptor-mediated endocytosis. Therefore, C/EBPβ was genetically fused to H22(scFv), which allows for CD64-mediated uptake of the fusion protein. H22(scFv)-C/EBPβ was cloned into a pMT vector (Fig. 7 and 8), transformed in *Escherichia coli* BL21(DE3), and expressed using the periplasmic stress expression protocol described by Barth et al. [21]. After one immobilized metal ion affinity chromatography (IMAC) purification step and size exclusion chromatography (SEC), the enrichment and identity of H22(scFv)-C/EBPβ were determined by SDS-PAGE followed by Coomassie brilliant blue staining and western blotting, respectively (Fig. 9a and 9b). Specific binding activity to murine peritoneal macrophages was confirmed by flow cytometry (Fig. 10). Modulation experiments revealed strongly down-regulated production of IL-12 in M1 macrophages mediated by H22(scFv)-C/EBPβ (Fig. 11). Additionally, C/EBPβ induced the production of anti-inflammatory IL-10 in M0 and M1 macrophages (Fig. 12). In contrast to IL-12, further pro-inflammatory cytokines, such as IL-6 (Fig. 13) showed decreased levels in M1 macrophages and increased levels in M0 and M2 macrophages TNF-α (Fig. 12 and 14). However, these cytokines are less appropriate soluble markers for M1 macrophages, as they are predominantly produced by T cells. On the level of surface receptors, H22(scFv)-C/EBPβ had minor effect on M1 macrophages. Interestingly, incubation of M2 macrophages with H22(scFv)-C/EBPβ resulted in a hybrid phenotype CD14^{high}/CD36^{high}/CD206^{high}/CD301^{low} (Fig. 14). By using CD64-mediated modulation of M1 macrophages a proof of principle was provided, of how to intervene in the disbalanced M1/M2 ratio during chronic inflammation.

### Potential indications for an M1-targeted therapy

The herein presented constructs could be applied in the following diseases: Rheumatoid arthritis, arthritis, inflammatory bowel disease, systemic lupus erythematosus, psoriasis, multiple sclerosis, diabetes type 1 and 2, atopic dermatitis, chronic wounds, cardiovascular diseases (e.g., atherosclerosis, acute myocardial infarction, peripheral vascular disease).

### Methods

### Ethics statement

The human skin biopsy was taken at the Sankt Franziskus Hospital in Aachen, Germany, in accordance with the guidelines of the ethical committee of University Hospital Aachen, Germany. The committee specifically approved this study. Written informed consent was obtained from the patient.

### Human patient material

We analyzed one skin biopsy specimen from a patient with atopic dermatitis. The specimen (4 mm in diameter) was taken from an inflamed region. The specimen was divided into four pieces and incubated in complete RPMI1640 medium supplemented with 10% fetal calf serum, 50 µg/ml penicillin and 100 µg/ml streptomycin (Invitrogen, Germany) to which was added 1 µM H22(scFv)-ETA' for 24 or 48 h. Control specimens were incubated for 24 h in complete medium only in order to determine the immunotoxin-independent death or extravasation of macrophages. One piece of the tissue was immediately frozen in liquid nitrogen and stored at -80°C prior to analysis by immunohistochemistry (here described as "untreated").

### Animals and SLS-induced cutaneous inflammation model

The animal experiments were approved by the local animal care and use review committee. All animals received human treatment in accordance with the requirements of the German Tierschutzgesetz, §8 Abs. 1 and with the guide for the care and use of laboratory animals published by the National Institutes of Health in 2011.One used > 6-week-old nude hairless transgenic male C57/BI6/SKH1-E mice expressing human CD64 (hCD64) in all experiments. This mouse model has been shown to closely parallel the situation in humans[22]. Chronic cutaneous inflammation was induced as described elsewhere [23]. Briefly, 5% (w/v) SLS in PBS was applied to a 1.5 x 1.5 cm skin surface area on both flanks of each mouse for 11 consecutive days. For immunotoxin administration, animals were anaesthetized with isofluran. Three intradermal injections of 20 µl 1 µM immunotoxin, or PBS control, were administered contralaterally. Finally, the animals were sacrificed and skin biopsies were taken, snap frozen in liquid nitrogen, and stored at -80°C prior to analysis.

### Isolation and in vitro polarization of macrophages

Peritoneal macrophages were induced in hCD64 transgenic mice by intraperitoneal injection of 1 ml 2% (w/v) BioGel P-100 (BioRad, Germany). After 3 days, mice were sacrificed and macrophages were isolated by peritoneal lavage using 5 ml of cold PBS (pH 7.4). After the lysis of red blood cells, macrophages were cultured at a concentration of 0.5-1.0 x 10⁶ cells/ml in complete medium in T75 tissue culture flasks for 2-4 h. Non-adherent cells were removed by washing with cold PBS and macrophages were detached by incubation with cold 0.5 mM EDTA in PBS (pH 7.4) for 10 min on ice. Appropriate numbers of cells were then seeded into assay plates and incubated overnight. The cells were then stimulated for 24 h with 100 U/ml IFN-γ (Peprotech, Germany) and 1 µg/ml LPS (Sigma-Aldrich, Germany) to generate M1 macrophages, or with 20 ng/ml IL-4 (Peprotech, Germany) for 24 h to generate M2 macrophages. Non-polarized macrophages were used as controls. Human monocytes were isolated from buffy coats by gradient centrifugation with Ficoll (VWR, Germany) and cultured overnight as described above. After the appropriate stimulus, cells were incubated for 72 h. Finally, stimuli were boosted with 50% of the initial amount for 24 h and macrophages were used in functional assays.

### In vitro plasticity of polarized macrophages

To study the plasticity of polarized macrophages, cells were exposed to the reverse stimulus for 24 h. Before repolarization, medium containing the initial stimulus was changed and cells were washed once with fresh medium. Surface expression and cytokine profiles were analyzed by flow cytometry and FlowCytomix, respectively.

### Flow cytometry

The expression of cell-surface receptors on macrophages was analyzed by flow cytometry. Cells were incubated with a fluorophore-labeled antibody in PBS (pH 7.4) containing 2 mM EDTA and 0.5% (w/v) BSA for 30 min on ice followed by two washes with PBS. The fluorescence was then analyzed on a FACSCalibur flow cytometer (Becton Dickinson, Germany). The following antibodies were used: mouse-anti-human CD64 [10.1]:FITC; rat-anti-mouse CD301:AlexaFluor488; rat-anti-mouse CD206:FITC; hamster-anti-mouse CD11c:RPE; rat-anti-mouse CD204:FITC (all from Serotec, Germany); mouse-anti-mouse CD284:PE; mouse-anti-human CD284: PE; rat-anti-mouse CD273: PE; mouse-anti-human CD273:PE; rat-anti-mouse CD14:FITC; rat-anti-mouse CD205:AlexaFluor488; mouse-anti-human CD200R:PE; mouse-anti-mouse MHCI:FITC; rat-anti-mouse MHCII:FITC; mouse-anti-human CD206: PE; rat-anti-mouse CD25:AlexaFluor488; rat-anti-mouse CD36:PE; rat-anti-mouse CD39:PE (all from eBioscience, Germany); mouse-anti-human CLEC10A/CD301:AlexaFluor488 (R&D Systems, Germany). Mouse-anti-rat IgG:FITC (eBioscience, Germany) was used as a secondary antibody for MOMA-1, MOMA-2 and ERTR-9 (kindly provided by Dr. Kraal, Amsterdam, Netherlands). The rat IgG2a K isotype control was labeled with PE or FITC (both eBioscience, Germany). Results are presented as relative fluorescence intensity calculated as follows: relative surface expression = (MFI_{antibody} - MFI_{isotype control})/MFI_{isotype} control. Comparisons between M1 and M2 polarized macrophages are presented as log(M1/M2) or log(M2/M1). All experiments were carried out in triplicate.

### Cytokine ELISA and FlowCytomix

The production of soluble cytokines such as IL-12, IL-6, TNF-α, IL-1α and IL-10 was measured by FlowCytomix according to the manufacturer's instructions (eBioscience, Germany). The concentration of murine RELM-α was determined by a sandwich enzyme-linked immunosorbent assay (ELISA). Therefore, high-binding 96-well flat-bottom microtiter plates (VWR, Germany) were coated with 1 µg/ml of rabbit-anti-mouse RELM-α capturing antibody (Peprotech, Germany) in PBS (pH 7.4) at 4°C overnight followed by blocking with 2% (w/v) BSA (Sigma, Germany) in PBS for 2 h at room temperature. All intermediate washing steps were carried out five times for 5 min using PBST (PBS + 0.05% Tween-20). Standard samples and supernatants were applied to the plate in a total volume of 100 µl and incubated at 4°C overnight. Captured cytokines were detected by incubating with 0.5 µg/ml of biotinylated rabbit-anti-mouse RELM-α (Peprotech, Germany) for 1 h at room temperature. Bound antibodies were detected using horseradish peroxidase (HRP)-conjugated streptavidin (Jackson ImmunoResearch, USA) at a concentration of 1 µg/ml for 30 min at room temperature. Finally, wells were extensively washed with PBST and incubated with TMB substrate (eBioscience, Germany) until color development was observed. The staining reaction was stopped with 1 M H₃PO₄ and the signal detected at E₄₅₀₋₅₇₀ₙₘ using an Epoch microplate spectrophotometer (Biotek, Germany). Data were analyzed using GraphPad Prism 5 software. All experiments were carried out in triplicate.

### Immunohistochemistry

Biopsy specimens were cut into 8-µm sections on a Cryostat CM 3050 (Leica, Germany) and mounted on superfrost slides (Menzel, Germany). After drying for 48-72 h, sections were fixed for 10 min with dry acetone and air-dried. Incubation with the primary antibody was carried out at 4°C overnight using mouse-anti-human CD64 [10.1]:FITC (eBioscience, Germany, 1:40), mouse-anti-human CD14-FITC (eBioscience, Germany, 1:40), rat-anti-mouse CD14-FITC (eBioscience, Germany, 1:40) in 1% (v/v) normal mouse serum in PBS, or mouse-anti-human CD206, mouse-anti-human CD301, rat-anti-mouse CD206, and rat-anti-mouse CD301 in 1% (v/v) normal goat serum in PBS. Slides were washed three times for 5 min with PBST and incubated with alkaline phosphatase (AP)-conjugated sheep anti-FITC (Southern Biotech, Germany, 1:400) in 1% (v/v) sheep serum in PBS (30 min) or AP-conjugated goat-anti-rat antibody (eBioscience, Germany, 1:400) in 1% (v/v) goat serum in PBS (30 min). After washing twice in PBST and once in Tris-HCI (0.1 M, pH 8.5), AP activity was detected using naphthol AS-BI phosphate (sodium salt, 50 mg/100 ml; Sigma, Germany) as the substrate and new fuchsin (10 mg/100 ml; Merck, USA) as the chromogen dissolved in 0.1 M Tris-HCI, pH 8.5, resulting in pink/red staining. Endogenous AP activity was inhibited by adding 0.35 mg/ml levamisole (Sigma, Germany) to the reaction mixture. Slides were lightly counterstained with hematoxylin.

### Cell viability assay

The cytotoxic effect of H22(scFv)-based immunotoxins or human cytolytic fusion proteins was assessed by measuring the conversion of XTT to a water-soluble orange formazan dye as previously described [24]. The concentration required to achieve a 50% reduction of protein synthesis (IC₅₀) relative to untreated control cells was calculated using GraphPad Prism 5 software. All experiments were carried out in triplicate. The three subpopulations (M1, M2 and non-polarized) showed diverse metabolic activity resulting in different scales of cell viability.

### Construction of open reading frames for PLTP and H22(scFv)-C/EBPβ

H22(scFv) was cloned in an open reading frame (ORF) together with different macrophage-modulatory proteins (MMPs). The ORF of C/EBPβ was modified with a 3' nuclear localization sequence (NLS) and with 5'-*Not*I and 3'-*Asc*I restriction sites by PCR followed by ligation into a *Not*I/*Asc*I-linearized pMT vector already containing the sequence for H22(scFv) (Fig. 23). Successful cloning was confirmed by test digestion and sequencing. The exemplified ORFs for MMPs are listed by their sequences. See list of sequences ID 1-17 in Fig. 27.

### Expression and purification of recombinant MMPs

MMPs were expressed in *Escherichia coli* BL21 (DE3) (*E. coli*) using the protocol for periplasmic stress expression in the presence of compatible solutes as described previously [21]. Briefly, after transformation bacteria were grown to an OD of 1.6 followed by stress induction with 500 mM D-sorbitol, 10 mM betaine monohydrate and 4% (w/w) NaCl. After an incubation of 30 min at 26°C with shaking (180 rpm), protein expression was induced by 2 mM IPTG. Bacteria were harvested 18 h after induction by centrifugation (4,000xg, 10 min, 4°C) and frozen at -80°C overnight. The frozen pellet was resuspended in preparation buffer (75 mM Tris-HCI, 300 mM NaCl, 5 mM DTT, 10 mM EDTA, 10% (v/w) glycerol, pH 8.0 containing a complete protease inhibitor cocktail (Roche, Germany)) at 4°C and sonicated 5 times for 60 sec at 200 W. Cell debris were removed by centrifugation (24,000xg, 20 min, 4°C) and for removal of EDTA protein preparation was dialyzed against PBS (pH 7.4) at 4°C overnight. MMPs were purified by immobilized metal-ion affinity chromatography (IMAC) using Ni-NTA Sepharose (Qiagen, Germany) and size exclusion chromatography (SEC) against PBS (pH 7.4) with Bio-Prep SE-100 /17 (Bio-Rad, Germany) columns according to the manufacturers' instructions.

### SDS-PAGE and Western Blot analysis of MMPs

Purity and quantity were assessed by sodium dodecylsulfate polyacrylamide gel electrophoresis (SDS-PAGE) and Western blot as described previously [17]. Protein concentration were quantified by densitometry using AIDA software after Coomassie staining in comparison with bovine serum albumin standards and verified by Bradford assays (Bio-Rad, Germany). Detection of MMPs on Western Blot was achieved using mouse-anti-Penta-His (1:5,000; Qiagen, Germany) in combination with an alkaline phosphatase-conjugated anti-mouse-IgG mAb (1:5,000; Sigma, Germany) followed by staining with Tris-HCI (pH 8.0) and 0.2 mg/ml naphtol-AS-Bi-phosphate (Sigma, Germany) supplemented with 1 mg/ml Fast-Red (Serva, Germany). Fig. 26 shows a 12% SDS-PAGE gel (A: Coomassie stained; B: Western Blot) of different MMPs after purification via IMAC and SEC. The gel contains: 1, PLTP; 2, H22(scFv)-C/EBPβ. Protein size is shown in kDa.

### Binding analysis of MMPs via flow cytometry

Cell binding activity of purified MMPs was assessed by flow cytometry. Binding was tested on murine peritoneal macrophages derived from hCD64-transgenic mice. Therefore, the cells were stimulated with IFN-γ (100 U/ml; Peprotech, Germany) 24 h prior to analysis. A total of 4x10⁵ cells were incubated with 50-100 nM of the MMP for 30 min on ice followed by washing with PBS containing 0.5% (w/v) BSA and 2 mM EDTA. For H22(scFv)-C/EBPβ, cells were stained with Penta-His-Alexa Fluor 488 Conjugate (1:100; Qiagen, Germany) for 30 min on ice (Fig. 11). Stained cells were washed twice as described above and analyzed by FACS Calibur (Becton Dickinson, Germany).

### Immunomodulation of polarized macrophages

Unpolarized murine macrophages were cultured in 12-well plates at a cell density of 3-5x10⁵ cells/ml with 100 nM H22(scFv)-C/EBPβ for 24 h followed by addition of the respective polarization stimuli. After a further incubation for 24 h, supernatants were collected for analysis of secreted cytokines. Finally, cells were detached using cold 0.5 mM EDTA in PBS (pH 7.4) for 10 min on ice and analyzed for expression of selected surface markers for M1 and M2 macrophages by flow cytometry.

### Statistical analysis

Statistical analysis was performed using GraphPad Prism 5 software. Data were expressed as the mean + standard deviation (SD) or standard error of the mean (SEM) as indicated. Statistical comparisons were made using an unpaired two-tailed Student's *t*-test, and one- or two-way ANOVA, where appropriate. *p≤0.05, **p≤0.01, ***p≤0.001.

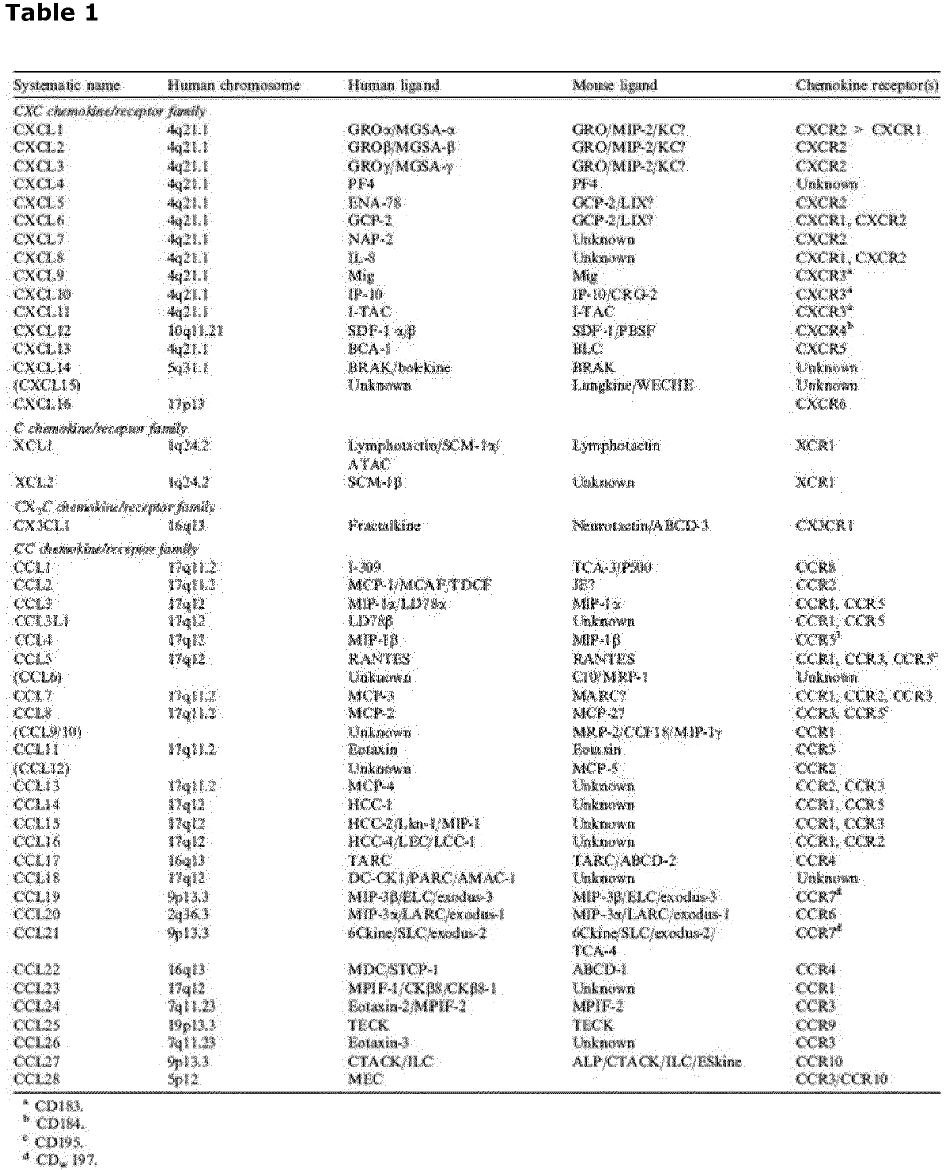

Extracted from R. Thorpe et al., Cytokine 21 (2003) 48-49.

**Table 2**

| **Name** | **Source** | **Target receptors** | **Target cells** | **Function** |
|---|---|---|---|---|
| IL-1 | macrophages, B cells, monocytes, dendritic cells | CD121a/IL1R1, CD121b/IL1R2 | T helper cells | co-stimulation |
| | | | B cells | Maturation & proliferation |
| | | | Nk cells | activation |
| | | | macrophages, endothelium, other | inflammation, small amounts induce acute phase reaction, large amounts induce fever |
| IL-2 | TH1-cells | CD25/IL2RA, CD122/IL2RB, CD132/IL2RG | activated T cells and B cells, NK cells, macrophages, oligodendrocytes | stimulates growth and differentiation of T cell response. Can be used in immunotherapy to treat cancer or suppressed for transplant patients. |
| IL-3 | activated T helper cells[3], mast cells, NK cells, endothelium, eosinophils | CD123/IL3RA, CD131/IL3RB | hematopoietic stem cells | growth and differentiation to e.g. erythrocytes, granulocytes |
| | | | mast cells | growth and histamine release |
| IL-4 | TH2-cells, just activated naive CD4+ cell, memory CD4+ cells, mast cells, macro phages | CD124/IL4R, CD132/IL2RG | activated B cells | proliferation and differentiation, IgG1 and IgE synthesis. Important role in allergic response (IgE) |
| | | | T cells | proliferation |
| IL-5 | TH2-cells, mast cells, eosinophils | CD125/IL5RA, CD131/IL3RB | eosinophils | production |
| | | | B cells | differentiation, IgA production |
| IL-6 | macrophages, TH2-cells, B cells, astrocytes, endothelium | CD126/IL6RA, CD130/IR6RB | activated B cells | differentiation into plasma cells |
| | | | plasma cells | antibody secretion |
| | | | hematopoietic stem cells | differentiation |
| | | | T cells, others | induces acute phase reaction, hematopoiesis, differentiation, inflammation |
| IL-7 | bone marrow stromal cells and thymus stromal cells | CD127/IL7RA, CD132/IL2RG | pre/pro-B cell, pre/pro-T cell, NK cells | involved in B, T, and NK cell survival, development, and homeostasis, ↑proinflammatory cytokines |
| IL-8 | macrophages, lymphocytes, epithelial cells, endothelial cells | CXCR1/IL8RA, CXCR2/IL8RB/CD12 8 | neutrophils, baso-phils, lymphocytes | Neutrophil chemotaxis |
| IL-9 | Th2-cells, specifically by CD4+ helper cells | CD129/IL9R | T cells, B cells | Potentiates IgM, IgG, IgE, stimulates mast cells |
| IL-10 | monocytes, TH2-cells, CD8+ T cells, mast cells, macrophages, B cell subset | CD210/IL10RA, CDW210B/IL10RB | macrophages | cytokine production |
| | | | B cells | activation |
| | | | Th1 cells | inhibits Th1 cytokine production (IFN-γ, TNF-β, IL-2) |
| | | | Th2 cells | Stimulation |
| IL-11 | bone marrow stroma | IL11RA | bone marrow stroma | acute phase protein production, osteoclast formation |
| IL-12 | dendritic cells, B cells, T cells, macrophages | CD212/IL12RB1, IR12RB2 | activated [3] T cells, | differentiation into Cytotoxic T cells with IL-2[3], ↑ IFN-γ, TNF-α, ↓ IL-10 |
| | | | NK cells | ↑ IFN-γ, TNF-α |
| IL-13 | activated TH2-cells, mast cells, NK cells | IL13R | TH2-cells, B cells, macro phages | Stimulates growth and differentiation of B-Cells (IgE), inhibits TH1-cells and the production of macrophage inflammatory cytokines (e.g. IL-1, IL-6), ↓ IL-8, IL-10, IL-12 |
| IL-14 | T cells and certain malignant B cells | | activated B cells | controls the growth and proliferation of B cells, inhibits Ig secretion |
| IL-15 | mononuclear phagocytes (and some other cells), especially macrophages following infection by virus(es) | IL15RA | T cells, activated B cells | Induces production of Natural Killer Cells |
| IL-16 | lymphocytes, epithelial cells, eosinophils, CD8+ T cells | CD4 | CD4+ T cells | CD4+ chemoattractant |
| IL-17 | subsets of T cells | CDw217/IL17RA, IL17RB | epithelium, endothelium, other | osteoclastogenesis, angiogenesis, ↑ inflammatory cytokines |
| IL-18 | macrophages | CDw218a/IL18Rl | Th1 cells, NK cells | Induces production of IFNγ, ↑ NK cell activity |
| IL-19 | - | IL20R | | - |
| IL-20 | - | IL20R | | regulates proliferation and differentiation of keratinocytes |
| IL-21 | - | IL21R | | |
| IL-22 | - | IL22R | | Activates STAT1 and STAT3 and increases production of acute phase proteins such as serum amyloid A, Alpha 1-antichymotrypsin and haptoglobin in hepatoma cell lines |
| IL-23 | - | IL23R | | Increases angiogenesis but reduces CD8 T-cell infiltration |
| IL-24 | - | IL20R | | Plays important roles in tumor suppression, wound healing and psoriasis by influencing cell survival. |
| IL-25 | - | LY6E | | Induces the production IL-4, IL-5 and IL-13, which stimulate eosinophil expansion |
| IL-26 | - | IL20R1 | | Enhances secretion of IL-10 and IL-8 and cell surface expression of CD54 on epithelial cells |
| IL-27 | - | IL27RA | | Regulates the activity of B lymphocyte and T lymphocytes |
| IL-28 | - | IL28R | | Plays a role in immune defense against viruses |
| IL-29 | - | | | Plays a role in host defenses against microbes |
| IL-30 | - | | | Forms one chain of IL-27 |
| IL-31 | - | IL31RA | | May play a role in inflammation of the skin |
| IL-32 | - | | | Induces monocytes and macrophages to secrete TNF-α, IL-8 and CXCL2 |
| IL-33 | - | | | Induces helper T cells to produce type 2 cytokine |
| IL-35 | regulatory T cells | | | Suppression of T helper cell activation |

### References

1. Gordon, S. and F.O. Martinez. Alternative activation of macrophages: mechanism and functions. Immunity, 2010. 32(5): p. 593-604.
2. Biswas, S.K. and A. Mantovani. Macrophage plasticity and interaction with lymphocyte subsets: cancer as a paradigm. Nat Immunol, 2010. 11(10): p. 889-96.
3. Cassetta, L., E. Cassol, and G. Poli. Macrophage polarization in health and disease. ScientificWorldJournal, 2011. 11: p. 2391-402.
4. Mantovani, A., A. Sica, S. Sozzani, P. Allavena, A. Vecchi, and M. Locati. The chemokine system in diverse forms of macrophage activation and polarization. Trends Immunol, 2004. 25(12): p. 677-86.
5. Mosser, D.M. and J.P. Edwards. Exploring the full spectrum of macrophage activation. Nat Rev Immunol, 2008. 8(12): p. 958-69.
6. Lee, S., S. Huen, H. Nishio, S. Nishio, H.K. Lee, B.S. Choi, C. Ruhrberg, and L.G. Cantley. Distinct macrophage phenotypes contribute to kidney injury and repair. J Am Soc Nephrol, 2011. 22(2): p. 317-26.
7. Murray, P.J. and T.A. Wynn. Protective and pathogenic functions of macrophage subsets. Nat Rev Immunol, 2011. 11(11): p. 723-37.
8. Lucas, T., A. Waisman, R. Ranjan, J. Roes, T. Krieg, W. Muller, A. Roers, and S.A. Eming. Differential roles of macrophages in diverse phases of skin repair. J Immunol, 2010. 184(7): p. 3964-77.
9. Odegaard, J.I. and A. Chawla. Pleiotropic actions of insulin resistance and inflammation in metabolic homeostasis. Science, 2013. 339(6116): p. 172-7.
10. Lumeng, C.N., J.L. Bodzin, and A.R. Saltiel. Obesity induces a phenotypic switch in adipose tissue macrophage polarization. J Clin Invest, 2007. 117(1): p. 175-84.
11. Xu, H., G.T. Barnes, Q. Yang, G. Tan, D. Yang, C.J. Chou, J. Sole, A. Nichols, J.S. Ross, L.A. Tartaglia, and H. Chen. Chronic inflammation in fat plays a crucial role in the development of obesity-related insulin resistance. J Clin Invest, 2003. 112(12): p. 1821-30.
12. Cardilo-Reis, L., S. Gruber, S.M. Schreier, M. Drechsler, N. Papac-Milicevic, C. Weber, O. Wagner, H. Stangl, O. Soehnlein, and C.J. Binder. Interleukin-13 protects from atherosclerosis and modulates plaque composition by skewing the macrophage phenotype. EMBO Mol Med, 2012. 4(10): p. 1072-86.
13. van Roon, J.A., A.J. van Vuuren, S. Wijngaarden, K.M. Jacobs, J.W. Bijlsma, F.P. Lafeber, T. Thepen, and J.G. van de Winkel. Selective elimination of synovial inflammatory macrophages in rheumatoid arthritis by an Fcgamma receptor I-directed immunotoxin. Arthritis Rheum, 2003. 48(5): p. 1229-38.
14. Liu, C., Y. Li, J. Yu, L. Feng, S. Hou, Y. Liu, M. Guo, Y. Xie, J. Meng, H. Zhang, B. Xiao, and C. Ma. Targeting the shift from m1 to m2 macrophages in experimental autoimmune encephalomyelitis mice treated with fasudil. PLoS One, 2013. 8(2): p. 13.
15. Orme, J. and C. Mohan. Macrophage subpopulations in systemic lupus erythematosus. Discov Med, 2012. 13(69): p. 151-8.
16. Crielaard, B.J., T. Lammers, M.E. Morgan, L. Chaabane, S. Carboni, B. Greco, P. Zaratin, A.D. Kraneveld, and G. Storm. Macrophages and liposomes in inflammatory disease: friends or foes? Int J Pharm, 2011. 416(2): p. 499-506.
17. Hetzel, C., C. Bachran, R. Fischer, H. Fuchs, S. Barth, and M. Stocker. Small cleavable adapters enhance the specific cytotoxicity of a humanized immunotoxin directed against CD64-positive cells. J Immunother, 2008. 31(4): p. 370-6.
18. Hetzel, C., C. Bachran, M.K. Tur, H. Fuchs, and M. Stocker. Improved immunotoxins with novel functional elements. Curr Pharm Des, 2009. 15(23): p. 2700-11.
19. Nathan, C. and A. Ding. Nonresolving inflammation. Cell, 2010. 140(6): p. 871-82.
20. Martinez, F.O., L. Helming, and S. Gordon. Alternative activation of macrophages: an immunologic functional perspective. Annu Rev Immunol, 2009. 27: p. 451-83.
21. Barth, S., M. Huhn, B. Matthey, A. Klimka, E.A. Galinski, and A. Engert. Compatible-solute-supported periplasmic expression of functional recombinant proteins under stress conditions. Appl Environ Microbiol, 2000. 66(4): p. 1572-9.
22. Heijnen, I.A. and J.G. Van de Winkel. A human Fc gamma RI/CD64 transgenic model for in vivo analysis of (bispecific) antibody therapeutics. J Hematother, 1995. 4(5): p. 351-6.
23. Thepen, T., A.J. van Vuuren, R.C. Kiekens, C.A. Damen, W.C. Vooijs, and J.G. van De Winkel. Resolution of cutaneous inflammation after local elimination of macrophages. Nat Biotechnol, 2000. 18(1): p. 48-51.
24. Huhn, M., S. Sasse, M.K. Tur, B. Matthey, T. Schinkothe, S.M. Rybak, S. Barth, and A. Engert. Human angiogenin fused to human CD30 ligand (Ang-CD30L) exhibits specific cytotoxicity against CD30-positive lymphoma. Cancer Res, 2001. 61(24): p. 8737-42.

## Claims

1. A human immunomodulatory fusion protein (IFP) for converting pro-inflammatory M1 macrophages to anti-inflammatory M2 like phenotypes of macrophages the IFP having at least one component A and at least one component B wherein
the component A is comprising or consisting of a binding domain for extra-cellular surface structures of a macrophage that internalizes said immunomodulatory fusion protein upon binding of component A of said immunomodulatory fusion protein, and
the component B is murine C/EBPβ, human C/EBPβ.

2. The immunomodulatory fusion protein of claim 1 wherein the component A of the immunomodulatory fusion protein is selected from the group of internalizing macrophage-specific cell surface receptor binding structures consisting of antibodies or their derivatives or fragments thereof, synthetic peptides such as scFv, mimotopes, etc. or chemical molecules such as carbohydrates, lipids, nucleic acids, peptides, vitamins, etc., and/or small molecules with up to 100 atoms with receptor-binding activity like ligands, in particular peptidic molecules, non-peptidic molecules, etc., and/or carbohydrate binding proteins and their ligands such as lectins, in particular calnexins, c-type lectins, I-type lectins, m-type lectins, p-type lectins, r-type lectins, galectins and their derivatives, and/or receptor binding molecules such as natural ligands to macrophage-specific cluster of differentiation (CD) antigens, like CD11c, CD14, CD64, etc., cytokines such as chemokines, colony stimulating factors, type-1 cytokines, type-2 cytokines, interferons, interleukins, lymphokines, monokines, etc., and/or adhesion molecules including their derivatives and mutants, and/or derivatives or combinations of any of the above listed binding structures.

3. The immunomodulatory fusion protein of claim 1 or 2 wherein component A is selected from the group consisting of CD64, CD11c, CD14, CD16b, CD25, CD36, CD39, CD80, CD86, CD89, CD273, CD284, HLA-A major histocompatibility complex, class I, A, HLA-B major histocompatibility complex, class I, B, HLA-C major histocompatibility complex, class I, C, HLA-E major histocompatibility complex, class I, E, HLA-F major histocompatibility complex, class I, F, HLA-G major histocompatibility complex, class I, G, HLA-DQA1 major histocompatibility complex, class II, DQ alpha 1, HLA-DQA2 major histocompatibility complex, class II, DQ alpha 2, HLA-DQB1 major histocompatibility complex, class II, DQ beta 1, HLA-DQB2 major histocompatibility complex, class II, DQ beta 2, HLA-DRA major histocompatibility complex, class II, DR alpha, HLA-DRB1 major histocompatibility complex, class II, DR beta 1, HLA-DRB3 major histocompatibility complex, class II, DR beta 3, HLA-DRB4 major histocompatibility complex, class II, DR beta 4, HLA-DRB5 major histocompatibility complex, class II, DR beta 5, CD74 molecule, major histocompatibility complex, class II invariant chain, HLA-DPA1 major histocompatibility complex, class II, DP alpha 1, HLA-DPB1 major histocompatibility complex, class II, DP beta 1, HLA-DMA major histocompatibility complex, class II, DM alpha, HLA-DMB major histocompatibility complex, class II, DM beta, HLA-DOA major histocompatibility complex, class II, DO alpha, HLA-DOB major histocompatibility complex, class II, DO beta.

4. The immunomodulatory fusion protein of any one of the claims 1 to 3 wherein component A is selected from the polypeptides of Seq ID Nos. 1-33.

5. The immunomodulatory fusion protein of any one of the claims 1 to 4 wherein the component A is a chemokine or a specifically binding fragment thereof, in particular
wherein component A is selected from the group consisting of CXC chemokine/receptor family such as CXCL1, CXCL2, CXCL3, CXCL4, CXCL5, CXCL6, CXCL7, CXCL8, CXCL9, CXCL10, CXCL11, CXCL12, CXCL13, CXCL14, KXCL15, CXCL16; or the C chemokine /receptor family, such as XCL1 and XCL2; or the CX₃C chemokine/receptor family, such as CX3CL1, or the CC chemokine/receptor family, such as CCL1, CCL2, CCL3, CCL3L1, CCL4, CCLS, (CCL6), CCL7, CCL8, (CCL9/10), CCL11, (CCL12)CCL13, CCL14, CCL15, CCL16, CCL17, CCL18, CCL19, CCL20, CCL21, CCL22, CCL23, CCL24, CCL25, CCL26, CCL27, and CCL28.

6. The immunomodulatory fusion protein of any one of the claims 1 to 5 wherein component A is an interleukin or a specifically binding fragment thereof in particular selected from the group consisting of IL-1; IL-2; IL-3; IL-4; IL-5; IL-6; IL-7; IL-8; IL-9; IL-10; IL-11; IL-12; IL-13; IL-14; IL-15; IL-16; IL-17; IL-18; IL-19; IL-20; IL-21; IL-22; IL-23; IL-24; IL-25; IL-26; IL-27; IL-28; IL-29; IL-30; IL-31; IL-32; IL-33; and IL-35.

7. The immunomodulatory fusion protein of anyone of the claims 1 to 6 selected from the group consisting of at least one protein having one of the amino acid sequences encoded by the polynucleotide of one of the Seq ID Nos. 34 - 44.

8. The immunomodulatory fusion protein of any one of the claims 1 to 7 wherein the components A and B are chemically coupled or fused to each other by genetic engineering, in particular protein having one of the amino acid sequences encoded by the polynucleotide of one of the Seq ID Nos. 45 - 49.

9. A process for manufacturing the immunomodulatory fusion protein of any one of the claims 1 to 8 comprising or consisting of the steps
- cloning the component B to yield a polynucleotide; and
- fuse said polynucleotide coding for component B with a polynucleotide coding for a protein of component A to yield a polynucleotide coding for the immunomodulatory fusion protein of the invention;
- expressing said polynucleotide coding for the immunomodulatory fusion protein of the invention in suitable host, such as *E. coli;*
- isolation and purification of the immunomodulatory fusion protein .

10. Polynucleotides encoding for the proteineous immunomodulatory fusion protein of any one of the claims 1 to 8.

11. A vector comprising or consisting of at least one polynucleotide of claim 10.

12. A cell having the vector of claim 11.

13. A medicament comprising or consisting of the immunomodulatory fusion protein of any one of the claims 1 to 8.

14. The immunomodulatory fusion protein of any one of the claims 1 to 8 for use in converting pro-inflammatory M1 macrophages in anti-inflammarory M2-like phenotype in the treatment of chronic inflammatory diseases.

15. An *ex vivo* method of converting pro-inflammatory M1 macrophage in an anti-inflammarory M2-like phenotype by contacting the M1 macrophage with the immunomodulatory fusion protein of at least one of the claims 1 to 8 for a sufficient time to effect the conversion of the M1 macrophage to the anti-inflammarory M2-like phenotype.

## Patentansprüche

1. Humanes immunmodulatorisches Fusionsprotein (IFP) zur Umwandlung von proinflammatorischen M1-Makrophagen in antiinflammatorische M2-artige Phänotypen von Makrophagen, wobei die IFP wenigstens eine Komponente A und wenigstens eine Komponente B aufweisen, wobei
Komponente A eine Bindungsdomäne für extrazelluläre Oberflächenstrukturen eines Makrophagen, die das immunmodulatorische Fusionsprotein nach der Bindung von Komponente A des immunmodulatorischen Fusionsproteins internalisiert, umfasst oder daraus besteht und
es sich bei Komponente B um murines C/EBPβ, humanes C/EBPβ handelt.

2. Immunmodulatorisches Fusionsprotein gemäß Anspruch 1, wobei die Komponente A des immunmodulatorischen Fusionsproteins ausgewählt ist aus der Gruppe der internalisierenden, makrophagenspezifischen Zelloberflächenrezeptor-Bindungsstrukturen, die aus Antikörpern oder ihren Derivaten oder Fragmenten davon bestehen, synthetischen Peptiden, wie scFv, Mimotope usw., oder chemischen Molekülen, wie Kohlenhydrate, Lipide, Nucleinsäuren, Peptide, Vitamine usw., und/oder kleinen Molekülen mit bis zu 100 Atomen mit rezeptorbindender Aktivität, wie Liganden, insbesondere peptidische Moleküle, nichtpeptidische Moleküle usw., und/oder kohlenhydratbindenden Proteinen und ihrer Liganden, wie Lektine, insbesondere Calnexine, Lektine des c-Typs, Lektine des I-Typs, Lektine des m-Typs, Lektine des p-Typs, Lektine des r-Typs, Galektine und ihre Derivate, und/oder rezeptorbindenden Molekülen, wie natürlichen Liganden von makrophagenspezifischen Cluster-of-Differentiation(CD)-Antigenen, wie CD11c, CD14, CD64 usw., Cytokinen, wie Chemokine, koloniestimulierenden Faktoren, Cytokine des Typs 1, Cytokine des Typs 2, Interferone, Interleukine, Lymphokine, Monokine usw., und/oder Adhäsionsmolekülen einschließlich ihrer Derivate und Mutanten und/oder Derivaten oder Kombinationen von einer der oben aufgeführten Bindungsstrukturen.

3. Immunmodulatorisches Fusionsprotein gemäß Anspruch 1 oder 2, wobei Komponente A aus der Gruppe ausgewählt ist, die aus CD64, CD11c, CD14, CD16b, CD25, CD36, CD39, CD80, CD86, CD89, CD273, CD284, HLA-A-Haupthistokompatibilitätskomplex, Klasse IA, HLA-B-Haupthistokompatibilitätskomplex, Klasse IB, HLA-C-Haupthistokompatibilitätskomplex, Klasse IC, HLA-E-Haupthistokompatibilitätskomplex, Klasse IE, HLA-F-Haupthistokompatibilitätskomplex, Klasse IF, HLA-G-Haupthistokompatibilitätskomplex, Klasse IG, HLA-DQA1-Haupthistokompatibilitätskomplex, Klasse II DQ alpha 1, HLA-DQA2-Haupthistokompatibilitätskomplex, Klasse II DQ alpha 2, HLA-DQB1-Haupthistokompatibilitätskomplex, Klasse II DQ beta 1, HLA-DQB2-Haupthistokompatibilitätskomplex, Klasse II DQ beta 2, HLA-DRA-Haupthistokompatibilitätskomplex, Klasse II DR alpha, HLA-DRB1-Haupthistokompatibilitätskomplex, Klasse II DR beta 1, HLA-DRB3-Haupthistokompatibilitätskomplex, Klasse II DR beta 3, HLA-DRB4-Haupthistokompatibilitätskomplex, Klasse II DR beta 4, HLA-DRB5-Haupthistokompatibilitätskomplex, Klasse II DR beta 5, CD74-Molekül, Haupthistokompatibilitätskomplex, Klasse II invariante Kette, HLA-DPA1-Haupthistokompatibilitätskomplex, Klasse II DP alpha 1, HLA-DPB1-Haupthistokompatibilitätskomplex, Klasse II DP beta 1, HLA-DMA-Haupthistokompatibilitätskomplex, Klasse II DM alpha, HLA-DMB-Haupthistokompatibilitätskomplex, Klasse II DM beta, HLA-DOA-Haupthistokompatibilitätskomplex, Klasse II DO alpha, HLA-DOB-Haupthistokompatibilitätskomplex, Klasse II DO beta besteht

4. Immunmodulatorisches Fusionsprotein gemäß einem der Ansprüche 1 bis 3, wobei Komponente A aus den Polypeptiden der SEQ ID Nr. 1-33 ausgewählt ist.

5. Immunmodulatorisches Fusionsprotein gemäß einem der Ansprüche 1 bis 4, wobei Komponente A ein Chemokin oder ein spezifisch bindendes Fragment davon ist,
wobei Komponente A insbesondere aus der Gruppe ausgewählt ist, die aus der CXC-Chemokin/Rezeptor-Familie, wie CXCL1, CXCL2, CXCL3, CXCL4, CXCL5, CXCL6, CXCL7, CXCL8, CXCL9, CXCL10, CXCL11, CXCL12, CXCL13, CXCL14, KXCL15, CXCL16; oder der C-Chemokin/Rezeptor-Familie, wie XCL1 und XCL2; oder der CX₃C-Chemokin/Rezeptor-Familie, wie CX3CL1, oder der CC-Chemokin/Rezeptor-Familie, wie CCL1, CCL2, CCL3, CCL3L1, CCL4, CCLS, (CCL6), CCL7, CCL8, (CCL9/10), CCL11, (CCL12)CCL13, CCL14, CCL15, CCL16, CCL17, CCL18, CCL19, CCL20, CCL21, CCL22, CCL23, CCL24, CCL25, CCL26, CCL27 und CCL28, besteht.

6. Immunmodulatorisches Fusionsprotein gemäß einem der Ansprüche 1 bis 5, wobei Komponente A ein Interleukin oder ein spezifisch bindendes Fragment davon ist,
wobei Komponente A insbesondere aus der Gruppe ausgewählt ist, die aus IL-1; IL-2; IL-3; IL-4; IL-5; IL-6; IL-7; IL-8; IL-9; IL-10; IL-11; IL-12; IL-13; IL-14; IL-15; IL-16; IL-17; IL-18; IL-19; IL-20; IL-21; IL-22; IL-23; IL-24; IL-25; IL-26; IL-27; IL-28; IL-29; IL-30; IL-31; IL-32; IL-33; und IL-35 besteht.

7. Immunmodulatorisches Fusionsprotein gemäß einem der Ansprüche 1 bis 6, das aus der Gruppe ausgewählt ist, die aus wenigstens einem Protein besteht, das eine der Aminosäuresequenzen aufweist, die von dem Polynucleotid gemäß einer der SEQ ID Nr. 34-44 codiert werden.

8. Immunmodulatorisches Fusionsprotein gemäß einem der Ansprüche 1 bis 7, wobei die Komponenten A und B durch gentechnische Methoden chemisch aneinander gekoppelt oder miteinander fusioniert sind, insbesondere ein Protein, das eine der Aminosäuresequenzen aufweist, die von dem Polynucleotid gemäß einer der SEQ ID Nr. 45-49 codiert werden.

9. Verfahren zur Herstellung des immunmodulatorischen Fusionsproteins gemäß einem der Ansprüche 1 bis 8, umfassend oder bestehend aus den Schritten:
- Klonieren der Komponente B unter Bildung eines Polynucleotids; und
- Fusionieren des Polynucleotids, das für Komponente B codiert, mit einem Polynucleotid, das für ein Protein von Komponente A codiert, unter Bildung eines Polynucleotids, das für das immunmodulatorische Fusionsprotein der Erfindung codiert;
- Exprimieren des Polynucleotids, das für das immunmodulatorische Fusionsprotein der Erfindung codiert, in einem geeigneten Wirt, wie *E. coli*;
- Isolieren und Reinigen des immunmodulatorischen Fusionsproteins.

10. Polynucleotide, die für das proteinartige immunmodulatorische Fusionsprotein gemäß einem der Ansprüche 1 bis 8 codieren.

11. Vektor, der wenigstens ein Polynucleotid gemäß Anspruch 10 umfasst oder daraus besteht.

12. Zelle, die den Vektor gemäß Anspruch 11 aufweist.

13. Medikament, das das immunmodulatorische Fusionsprotein gemäß einem der Ansprüche 1 bis 8 umfasst oder daraus besteht.

14. Immunmodulatorisches Fusionsprotein gemäß einem der Ansprüche 1 bis 8 zur Verwendung bei der Umwandlung von proinflammatorischen M1-Makrophagen in antiinflammatorische M2-artige Phänotypen bei der Behandlung von chronischen Entzündungserkrankungen.

15. Ex-vivo-Verfahren zur Umwandlung eines proinflammatorischen M1-Makrophagen in einen antiinflammatorischen M2-artigen Phänotyp durch In-Kontakt-Bringen des M1-Makrophagen mit dem immunmodulatorischen Fusionsprotein gemäß wenigstens einem der Ansprüche 1 bis 8 während einer ausreichenden Zeitdauer, um die Umwandlung des M1-Makrophagen in den antiinflammatorischen M2-artigen Phänotyp zu bewirken.

## Revendications

1. Protéine de fusion immunomodulatrice (IFP, « *Immunomodulatory fusion protein* ») humaine pour convertir des macrophages pro-inflammatoires M1 en phénotypes de macrophage anti-inflammatoires semblables à des M2, l'IFP ayant au moins un composant A et au moins un composant B dans lesquels
le composant A comprend ou consiste en un domaine de liaison pour des structures de surface extracellulaires d'un macrophage qui internalise ladite protéine de fusion immunomodulatrice lors de la liaison du composant A de ladite protéine de fusion immunomodulatrice, et
le composant B est la C/EBPβ murine, la C/EBPβ humaine.

2. Protéine de fusion immunomodulatrice selon la revendication 1, dans laquelle le composant A de la protéine de fusion immunomodulatrice est choisi dans le groupe des structures de liaison aux récepteurs de surface cellulaire spécifiques aux macrophages d'internalisation consistant en des anticorps ou leurs dérivés ou fragments de ceux-ci, des peptides de synthèse tels que scFv, des mimotopes, etc. ou des molécules chimiques telles que les glucides, les lipides, les acides nucléiques, les peptides, les vitamines, etc., et/ou de petites molécules avec jusqu'à 100 atomes avec une activité de liaison au récepteur comme les ligands, en particulier des molécules peptidiques, des molécules non peptidiques, etc., et/ou des protéines de liaison aux glucides et leurs ligands tels que les lectines, en particulier les calnexines, les lectines de type c, les lectines de type 1, les lectines de type m, les lectines de type p, les lectines de type r, les galectines et leurs dérivés, et/ou des molécules de liaison au récepteur telles que des ligands naturels à des antigènes de classes de différenciation (CD, « *cluster of differentiation* ») spécifiques aux macrophages, comme CD11c, CD14, CD64, etc., des cytokines telles que des chimiokines, des facteurs de stimulation de colonies, les cytokines de type 1, les cytokines de type 2, les interférons, les interleukines, les lymphokines, les monokines, etc., et/ou des molécules d'adhésion, y compris leurs dérivés et mutants, et/ou des dérivés ou des combinaisons de l'une quelconque des structures de liaison énumérées ci-dessus.

3. Protéine de fusion immunomodulatrice selon la revendication 1 ou 2, dans laquelle le composant A est choisi dans le groupe constitué par CD64, CD11c, CD14, CD16b, CD25, CD36, CD39, CD80, CD86, CD89, CD273, CD284, le complexe majeur d'histocompatibilité HLA-A, Classe I, A, le complexe majeur d'histocompatibilité HLA-B, classe I, B, le complexe majeur d'histocompatibilité HLA-C, classe I, C, le complexe majeur d'histocompatibilité HLA-E, classe I, E, le complexe majeur d'histocompatibilité HLA-F, classe I, F, le complexe majeur d'histocompatibilité HLA-G, classe I, G, le complexe majeur d'histocompatibilité HLA-DQA1, classe II, DQ alpha 1, le complexe majeur d'histocompatibilité HLA-DQA2, classe II, DQ alpha 2, le complexe majeur d'histocompatibilité HLA-DQB1, Classe II, DQ bêta 1, le complexe majeur d'histocompatibilité HLA-DQB2, classe II, DQ bêta 2, le complexe majeur d'histocompatibilité HLA-DRA, classe II, DR alpha, le complexe majeur d'histocompatibilité HLA-DRB1, classe II, DR bêta 1, le complexe majeur d'histocompatibilité HLA-DRB3, classe II, DR bêta 3, le complexe majeur d'histocompatibilité HLA-DRB4, classe II, DR bêta 4, le complexe majeur d'histocompatibilité HLA-DRB5, classe II, DR bêta 5, la molécule CD74, complexe majeur d'histocompatibilité, chaîne invariante de classe II, le complexe majeur d'histocompatibilité HLA-DPA1, classe II, DP alpha 1, le complexe majeur d'histocompatibilité HLA-DPB1, classe II, DP bêta 1, le complexe majeur d'histocompatibilité HLA-DMA, classe II, DM alpha, le complexe majeur d'histocompatibilité HLA-DMB, classe II, DM bêta, le complexe majeur d'histocompatibilité HLA-DOA, classe II, DO alpha, le complexe majeur d'histocompatibilité HLA-DOB, classe II, DO bêta.

4. Protéine de fusion immunomodulatrice selon l'une quelconque des revendications 1 à 3, dans laquelle le composant A est choisi parmi les polypeptides de Seq ID N° 1-33.

5. Protéine de fusion immunomodulatrice selon l'une quelconque des revendications 1 à 4, dans laquelle le composant A est une chimiokine ou un fragment spécifiquement liant de celle-ci, en particulier
dans laquelle le composant A est choisi dans le groupe consistant en une famille de chimiokine/récepteur CXC telle que CXCL1, CXCL2, CXCL3, CXCL4, CXCL5, CXCL6, CXCL7, CXCL8, CXCL9, CXCL10, CXCL11, CXCL12, CXCL13, CXCL14, KXCL15, CXCL16 ; ou la famille de chimiokine/récepteur C, telle que XCL1 et XCL2 ; ou la famille de chimiokine/récepteur CX₃C, telle que CX3CL1, ou la famille de chimiokine/récepteur CC, telle que CCL1, CCL2, CCL3, CCL3L1, CCL4, CCLS, (CCL6), CCL7, CCL8, (CCL9/10), CCL11, (CCL12) CCL13, CCL14, CCL15, CCL16, CCL17, CCL18, CCL19, CCL20, CCL21, CCL22, CCL23, CCL24, CCL25, CCL26, CCL27, et CCL28.

6. Protéine de fusion immunomodulatrice selon l'une quelconque des revendications 1 à 5, dans laquelle le composant A est une interleukine ou un fragment spécifiquement liant de celle-ci en particulier choisi dans le groupe constitué par IL-1 ; IL-2 ; IL-3 ; IL-4 ; IL-5 ; IL-6 ; IL-7 ; IL-8 ; IL-9 ; IL-10 ; IL-11 ; IL-12 ; IL-13 ; IL-14 ; IL-15 ; IL-16 ; IL-17 ; IL-18 ; IL-19 ; IL-20 ; IL-21 ; IL-22 ; IL-23 ; IL-24 ; IL-25 ; IL-26 ; IL-27 ; IL-28 ; IL-29 ; IL-30 ; IL-31 ; IL-32 ; IL-33 ; et IL-35.

7. Protéine de fusion immunomodulatrice selon l'une quelconque des revendications 1 à 6, choisie dans le groupe constitué par au moins une protéine ayant l'une des séquences d'acides aminés codées par le polynucléotide de l'une des Seq. ID N° 34 - 44.

8. Protéine de fusion immunomodulatrice selon l'une quelconque des revendications 1 à 7, dans laquelle les composants A et B sont chimiquement couplés ou fusionnés l'un à l'autre par génie génétique, en particulier une protéine ayant l'une des séquences d'acides aminés codées par le polynucléotide de l'une des Seq ID N° 45 - 49.

9. Procédé de fabrication de la protéine de fusion immunomodulatrice de l'une quelconque des revendications 1 à 8 comprenant ou consistant en les étapes de
- clonage du composant B pour donner un polynucléotide ; et de
- fusion dudit polynucléotide codant pour le composant B avec un polynucléotide codant pour une protéine du composant A pour donner un polynucléotide codant pour la protéine de fusion immunomodulatrice de l'invention ;
- expression dudit polynucléotide codant pour la protéine de fusion immunomodulatrice de l'invention dans un hôte approprié, tel que *E. coli* ;
- isolement et purification de la protéine de fusion immunomodulatrice.

10. Polynucléotides codant pour la protéine de fusion immunomodulatrice protéineuse de l'une quelconque des revendications 1 à 8.

11. Vecteur comprenant ou consistant en au moins un polynucléotide de la revendication 10.

12. Cellule ayant le vecteur de la revendication 11.

13. Médicament comprenant ou consistant en la protéine de fusion immunomodulatrice de l'une quelconque des revendications 1 à 8.

14. Protéine de fusion immunomodulatrice selon l'une quelconque des revendications 1 à 8 pour utilisation dans la conversion de macrophages pro-inflammatoires M1 en phénotype anti-inflammatoire semblable aux M2 dans le traitement de maladies inflammatoires chroniques.

15. Procédé *ex vivo* de conversion de macrophage pro-inflammatoire M1 en un phénotype anti-inflammatoire semblable à des M2 par mise en contact du macrophage M1 avec la protéine de fusion immunomodulatrice d'au moins l'une des revendications 1 à 8 pendant un temps suffisant pour effectuer la conversion du macrophage M1 en le phénotype anti-inflammatoire semblable à des M2.
